(19) **Europäisches Patentamt / European Patent Office / Office européen des brevets**

(11) **EP 4 737 480 A1**

(12) **EUROPEAN PATENT APPLICATION**
published in accordance with Art. 153(4) EPC

(43) Date of publication:
06.05.2026 Bulletin 2026/19

(21) Application number: 24832082.2

(22) Date of filing: 28.06.2024

(51) International Patent Classification (IPC):
C07K 16/28 (2006.01)    A61K 39/395 (2006.01)
A61K 47/64 (2017.01)    A61P 35/00 (2006.01)
A61P 43/00 (2006.01)    C12N 9/50 (2006.01)
C12N 15/13 (2006.01)    C12N 15/57 (2006.01)
C12P 21/08 (2006.01)

(52) Cooperative Patent Classification (CPC):
A61K 39/395; A61K 47/64; A61P 35/00;
A61P 43/00; C07K 16/00; C07K 16/28; C12N 9/48;
C12N 9/50

(86) International application number:
PCT/JP2024/023474

(87) International publication number:
WO 2025/005224 (02.01.2025 Gazette 2025/01)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 28.06.2023 JP 2023106329

(71) Applicant: Japan Science and Technology Agency
Kawaguchi-shi, Saitama 332-0012 (JP)

(72) Inventors:
• HIFUMI, Emi
Oita-shi, Oita 870-1103 (JP)
• UDA, Taizo
Oita-shi, Oita 870-0045 (JP)

(74) Representative: Wächtershäuser & Hartz
Patentanwaltspartnerschaft mbB
Weinstraße 8
80333 München (DE)

Remarks:
The complete document including Reference Table(s) and the Sequence Listing(s) can be downloaded from the EPO website

(54) **POLYPEPTIDE DERIVED FROM ANTIBODY LIGHT-CHAIN HAVING HER2 DECOMPOSITION ACTIVITY, HER2-BINDING POLYPEPTIDE COMPRISING SAID POLYPEPTIDE, METHODS FOR PRODUCING SAID POLYPEPTIDES, AND USE OF SAID POLYPEPTIDES**

(57) The present invention provides an antigenase obtained by imparting high enzymatic activity to an antibody having a high anticancer effect. The present invention relates to a polypeptide including hypervariable regions derived from an anti-HER2 antibody light chain, as described in (1a) or the like: (1a) a polypeptide consisting of an amino acid sequence that includes a CDR1 region consisting of an amino acid sequence set forth in SEQ ID NO: 1, a CDR2 region consisting of an amino acid sequence set forth in SEQ ID NO: 2, and a CDR3 region consisting of an amino acid sequence set forth in SEQ ID NO: 3, and has an aspartic acid residue at position 70 of a variable region in accordance with the Kabat numbering scheme, wherein proline residues at positions 95 and 96 of a variable region in accordance with the Kabat numbering scheme are deleted or substituted with amino acid residues other than proline residues.

EP 4 737 480 A1

**Description**

Technical Field

The present invention relates to a polypeptide derived from an anti-HER2 antibody light chain having HER2 decomposition activity, a HER2-binding polypeptide comprising the polypeptide, a method for producing the same, and an anticancer agent containing the same.

Background Art

An antibody is composed of a heavy chain (H chain) and a light chain (L chain). The heavy chain and the light chain are each composed of a variable region (VR) and a constant region (CR), the variable region including hypervariable regions (CDRs: complementarity-determining regions).

In recent years, an antibody having enzyme-like activity, that is, an abzyme, has attracted considerable attention. Such an abzyme is expected to be applied in many fields, including the medical field, the chemical industry, and the food industry, because it has both high molecular recognition ability of an antibody and enzymatic activity. In particular, an abzyme that has high specificity to a target molecule and is capable of exhibiting cytotoxicity against the target molecule by enzymatic activity is expected to be used as an effective anticancer drug with few side effects. Particularly, since a human abzyme is expected to cause few side effects when administered to the human body, domestic and foreign pharmaceutical companies have been waiting for the development of useful human abzymes.

The present inventors have conducted various studies on abzymes (see, for example, Patent Literatures 1 to 4).

In order to clinically use an abzyme as a pharmaceutical product, it is important to stably mass-produce an abzyme having sufficient activity. However, many abzymes exhibit insufficient enzymatic activity. When the abzymes are artificially synthesized by an intracellular or extracellular expression system using a genetic recombination technique, there is a problem of unstable performance and significant lot-to-lot variation.

Therefore, the present inventors have conducted various studies to produce an abzyme composed of an antibody light chain having higher enzymatic activity and reported that, in an amino acid sequence of an antibody light chain, deletion or substitution of the 95th proline residue in a variable region in accordance with the Kabat numbering scheme enables the light chain to form the steric conformation of catalytic triad residues (D1/S27/H93), thereby yielding an antibody light chain (antigenase) with enhanced enzymatic activity (Patent Literature 5).

Citation List

Patent Literature

Patent Literature 1: JP-A-2006-197930
Patent Literature 2: WO 2011/102517
Patent Literature 3: WO 2013/133253
Patent Literature 4: WO 2015/025786
Patent Literature 5: WO 2021/015237

Summary of Invention

Technical Problem

It is an object of the present invention to provide an antigenase obtained by imparting high enzymatic activity to an antibody having a high anticancer effect.

Solution to Problem

As a result of studies for imparting enzymatic activity to an anti-HER2 antibody light chain having an excellent anticancer effect against cancers that overexpress HER2, such as breast cancer, deletion of a proline residue at position 95 of the variable region has been considered insufficient to impart enzymatic activity to the light chain, since it lacks S27a/H93 described in Patent Literature 5.

However, the present inventors have found that simultaneous deletion of the proline residues at positions 95 and 96 of the variable region of the anti-HER2 antibody light chain unexpectedly yields a polypeptide derived from the anti-HER2 antibody light chain having enzymatic activity close to 4 times that of the light chain, and also that the use of the polypeptide enables the development of a novel HER2-targeted therapeutic agent having, in addition to HER2 decomposition activity, one or more activities selected from the group consisting of antibody-dependent cellular cytotoxicity, complement-dependent cytotoxicity, and neutralizing activity. Thus, the present invention has been accomplished.

Accordingly, the present invention provides [1] to [14] below.

[1] A polypeptide derived from an anti-HER2 antibody light chain, wherein a hypervariable region is selected from the group consisting of (1a) to (1c):

(1a) a polypeptide consisting of an amino acid sequence that includes a CDR1 region consisting of an amino acid sequence set forth in SEQ ID NO: 1, a CDR2 region consisting of an amino acid sequence set forth in SEQ ID NO: 2, and a CDR3 region consisting of an amino acid sequence set forth in SEQ ID NO: 3, and has an aspartic acid residue at position 70 of a variable region in accordance with the Kabat numbering scheme, wherein proline residues at positions 95 and 96 of the variable region in accordance with the Kabat numbering scheme are deleted or substituted with amino acid residues other than proline residues;
(1b) a polypeptide consisting of an amino acid sequence that includes a CDR1 region consisting of an amino acid sequence in which one or several amino acids are substituted, added, or deleted in the amino acid sequence set forth in SEQ ID NO: 1, a CDR2 region consisting of an amino acid sequence in which one or several amino acids are substituted, added, or deleted in the amino acid sequence set forth in SEQ ID NO: 2, and a CDR3 region consisting of an amino acid sequence set forth in SEQ ID NO: 3, and has an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues; and
(1c) a polypeptide consisting of an amino acid sequence that includes a CDR1 region consisting of an amino acid sequence having 90% or more identity with the amino acid sequence set forth in SEQ ID NO: 1, a CDR2 region consisting of an amino acid sequence having 90% or more identity with the amino acid sequence set forth in SEQ ID NO: 2, and a CDR3 region consisting of an amino acid sequence set forth in SEQ ID NO: 3, and has an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues.

[2] A polypeptide derived from an anti-HER2 antibody light chain, wherein a variable region is a polypeptide selected from the group consisting of (2a) to (2c):

(2a) a polypeptide consisting of an amino acid sequence set forth in SEQ ID NO: 4;
(2b) a polypeptide consisting of an amino acid sequence in which one or several amino acids are substituted, added, or deleted in the amino acid sequence set forth in SEQ ID NO: 4, the amino acid sequence having an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues; and
(2c) a polypeptide consisting of an amino acid sequence having 90% or more identity with the amino acid sequence set forth in SEQ ID NO: 4, the amino acid sequence having an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues.

[3] The polypeptide according to [1] or [2], further comprising a variable region of an anti-HER2 antibody heavy chain as set forth in SEQ ID NO: 12.
[4] The polypeptide according to [3], wherein the hypervariable region of an anti-HER2 antibody light chain according to [1] and the variable region of an anti-HER2 antibody heavy chain as set forth in SEQ ID NO: 12, or the variable region of an anti-HER2 antibody light chain according to [2] and the variable region of an anti-HER2 antibody heavy chain as set forth in SEQ ID NO: 12, are linked via a linker.
[5] The polypeptide according to [4], further comprising an Fc region linked thereto.
[6] The polypeptide according to [3], wherein

a light-chain constant region linked to the hypervariable region of an anti-HER2 antibody according to [1] and a heavy-chain CH1 region linked to a heavy-chain variable region set forth in SEQ ID NO: 12 are associated through a disulfide bond; or

a light-chain constant region linked to the variable region of an anti-HER2 antibody according to[2] and a heavy-chain CH1 region linked to a heavy-chain variable region set forth in SEQ ID NO: 12 are associated through a disulfide bond.

[7] A polypeptide comprising a heavy-chain CH2 and a heavy-chain CH3 that are linked to the CH1 region of the polypeptide according to [6].

[8] A method for producing a polypeptide derived from an anti-HER2 antibody light chain having enzymatic activity or improved enzymatic activity, the method comprising:

a modification step of modifying a polynucleotide encoding an anti-HER2 antibody light chain in which a hypervariable region is (1a) a polypeptide comprising a CDR1 region consisting of an amino acid sequence set forth in SEQ ID NO: 1, a CDR2 region consisting of an amino acid sequence set forth in SEQ ID NO: 2, and a CDR3 region consisting of an amino acid sequence set forth in SEQ ID NO: 3, and having an aspartic acid residue at position 70 of a variable region in accordance with the Kabat numbering scheme; (1b) a polypeptide comprising a CDR1 region consisting of an amino acid sequence in which one or several amino acids are substituted, added, or deleted in the amino acid sequence set forth in SEQ ID NO: 1, a CDR2 region consisting of an amino acid sequence in which one or several amino acids are substituted, added, or deleted in the amino acid sequence set forth in SEQ ID NO: 2, and a CDR3 region consisting of an amino acid sequence set forth in SEQ ID NO: 3, and having an aspartic acid residue at position 70 of a variable region in accordance with the Kabat numbering scheme; or (1c) a polypeptide comprising a CDR1 region consisting of an amino acid sequence having 90% or more identity with the amino acid sequence set forth in SEQ ID NO: 1, a CDR2 region consisting of an amino acid sequence having 90% or more identity with the amino acid sequence set forth in SEQ ID NO: 2, and a CDR3 region consisting of an amino acid sequence set forth in SEQ ID NO: 3, and having an aspartic acid residue at position 70 of a variable region in accordance with the Kabat numbering scheme, wherein the polypeptide consists of an amino acid sequence in which amino acid residues at positions 95 and 96 of the variable region in accordance with the Kabat numbering scheme are proline residues, so that the proline residues are deleted or substituted to give a polynucleotide encoding a polypeptide derived from an anti-HER2 antibody light chain, wherein the variable region is a polypeptide consisting of an amino acid sequence in which amino acid residues at positions 95 and 96 of the variable region in accordance with the Kabat numbering scheme are deleted or substituted with amino acid residues other than proline residues; and
an expression step of expressing a polypeptide derived from an anti-HER2 antibody light chain having enzymatic activity, by using an expression vector comprising a polynucleotide encoding an anti-HER2 antibody light chain after the modification step, in an intracellular or extracellular expression system.

[9] A method for producing a polypeptide derived from an anti-HER2 antibody light chain having enzymatic activity or improved enzymatic activity, the method comprising:

a modification step of modifying a polynucleotide encoding a polypeptide derived from an anti-HER2 antibody light chain in which a variable region is (2a) a polypeptide consisting of an amino acid sequence set forth in SEQ ID NO: 4; (2b) a polypeptide consisting of an amino acid sequence in which one or several amino acids are substituted, added, or deleted in the amino acid sequence set forth in SEQ ID NO: 4, the amino acid sequence having an aspartic acid residue at position 70 of a variable region in accordance with the Kabat numbering scheme; or (2c) a polypeptide having 90% or more identity with the amino acid sequence set forth in SEQ ID NO: 4 and having an aspartic acid residue at position 70 of a variable region in accordance with the Kabat numbering scheme, wherein the polypeptide consists of an amino acid sequence in which amino acid residues at positions 95 and 96 of the variable region in accordance with the Kabat numbering scheme are proline residues, so that the proline residues are deleted or substituted to give a polynucleotide encoding a polypeptide derived from an anti-HER2 antibody light chain, wherein the variable region is a polypeptide consisting of an amino acid sequence in which amino acid residues at positions 95 and 96 of the variable region in accordance with the Kabat numbering scheme are deleted or substituted with amino acid residues other than proline residues; and
an expression step of expressing a polypeptide derived from an anti-HER2 antibody light chain having enzymatic activity, by using an expression vector comprising a polynucleotide encoding an anti-HER2 antibody light chain after the modification step, in an intracellular or extracellular expression system.

[10] An anticancer agent comprising a component selected from the group consisting of: the polypeptide derived from an anti-HER2 antibody light chain according to [1] or [2]; a HER2-binding polypeptide comprising the polypeptide derived from the light chain; and a functional fragment of the HER2-binding polypeptide comprising the polypeptide derived from the light chain.

[11] A pharmaceutical composition for treating cancer, comprising a component selected from the group consisting of: the polypeptide derived from an anti-HER2 antibody light chain according to [1] or [2]; an anti-HER2 antibody comprising the polypeptide derived from the light chain; and a functional fragment of a HER2-binding polypeptide comprising the polypeptide derived from the light chain.

[12] Use of a component for production of an anticancer agent, the component being selected from the group consisting of: the polypeptide derived from an anti-HER2 antibody light chain according to [1] or [2]; a HER2-binding polypeptide comprising the polypeptide derived from the light chain; and a functional fragment of the HER2-binding polypeptide comprising the polypeptide derived from the light chain.

[13] A component for use in treatment of cancer, the component being selected from the group consisting of: the polypeptide derived from an anti-HER2 antibody light chain according to [1] or [2]; a HER2-binding polypeptide comprising the polypeptide derived from the light chain; and a functional fragment of an anti-HER2 antibody comprising the polypeptide derived from the light chain.

[14] A method for treating cancer, the method comprising administering a component selected from the group consisting of: the polypeptide derived from an anti-HER2 antibody light chain according to [1] or [2]; a HER2-binding polypeptide comprising the polypeptide derived from the light chain; and a functional fragment of the HER2-binding polypeptide comprising the polypeptide derived from the light chain.

Advantageous Effects of Invention

Since the polypeptide derived from the anti-HER2 antibody light chain according to the present invention has excellent enzymatic activity for decomposition of HER2 peptides, a component selected from the group consisting of the polypeptide derived from the anti-HER2 antibody light chain, a HER2-binding polypeptide comprising the polypeptide derived from the light chain, and a functional fragment of the HER2-binding polypeptide comprising the polypeptide derived from the light chain is useful as an anticancer agent targeting HER2, the anticancer agent having, in addition to HER2 decomposition activity, one or more activities selected from the group consisting of antibody-dependent cellular cytotoxicity, complement-dependent cytotoxicity, and neutralizing activity.

Brief Description of Drawings

Figure 1 illustrates a base sequence around P95P96 of a trastuzumab light-chain gene.

Figure 2A shows results of SDS-PAGE of a trastuzumab light chain.

Figure 2B shows results of SDS-PAGE of a trastuzumab light chain P95 ΔP96Δ.

Figure 2C shows result of SDS-PAGE of a polypeptide in which a scFv trastuzumab light-chain variable region P95ΔP96Δ, a linker, and a heavy chain variable region are joined.

Figure 3 illustrates time-dependent changes in decomposition activity of a trastuzumab light chain, a trastuzumab light chain P95ΔP96Δ, and a scFv trastuzumab light chain P95ΔP96Δ against trypsin-like substrate molecules, Arg-pNA substrates (R-pNA).

Figure 4 illustrates synthetic design diagrams of FRET-HER2-1 (a) and FRET-HER2-2 (b) peptides.

Figure 5 illustrates cleaved sites of FRET-HER2 peptide-1 (579-600) and structures of decomposition products thereof.

Figure 6 illustrates time-dependent changes in decomposition activity against trastuzumab light chain (Tmb-L), trastuzumab light chain P95ΔP96Δ (Tmb-L-PΔΔ), scFv trastuzumab light chain (Tmb-scFv), scFv trastuzumab light chain P95ΔP96Δ (Tmb-scFv), and trastuzumab FRET-HER2 peptide-1.

Figure 7 illustrates an amino acid sequence of a variable region of a trastuzumab light chain.

Figure 8 illustrates cleaved sites of FRET-HER2 peptide-2 (Loop2 + Loop3) and structures of decomposition products thereof.

Figure 9 illustrates time-dependent changes in decomposition activity against trastuzumab light chain (Tmb-L), trastuzumab light chain P95ΔP96Δ (Tmb-L-PΔΔ), scFv trastuzumab light chain (Tmb-scFv), scFv trastuzumab light chain P95ΔP96Δ (Tmb-scFv), and trastuzumab FRET-HER2 peptide-2.

Figure 10 shows results of calculation of kinetic constants by Lineweaver-Burk plot for trastuzumab light chain P95ΔP96Δ and scFv trastuzumab light chain P95ΔP96Δ reaction.

Figure 11 illustrates an amino acid sequence of #4 polypeptide.

Figure 12 shows results of conformational simulation of a trastuzumab light chain (left) and a trastuzumab light chain P95ΔP96Δ (right).

Figure 13 shows results of cytotoxicity assay of trastuzumab (Trasutuzumab in the figure), trastuzumab light chain P95ΔP96Δ (Tmb-L-PPΔ in the figure), scFv trastuzumab light chain(Tmb-scFv in the figure), scFv trastuzumab light chain P95ΔP96Δ (Tmb-ScFv-PPΔ in the figure), and anti-HER2 antibody light chain (Tmb-L in the figure) against the human breast cancer cell line BT-474.

Figure 14 shows results of cytotoxicity assay (bar graph) of trastuzumab (Trasutuzumab in the figure), trastuzumab light chain P95ΔP96Δ (Tmb-L-PPΔ in the figure), scFv trastuzumab light chain(Tmb-scFv in the figure), scFv trastuzumab light chain P95ΔP96Δ (Tmb-ScFv-PPΔ in the figure), and anti-HER2 antibody light chain (Tmb-L in the figure) against the human breast cancer cell line BT-474.

Figure 15 illustrates relative values in the antigen decomposition activity assay of trastuzumab, a scFv thereof (trastuzumab-scFv, and trastuzumab-scFv-PPΔ lacking Pro95 and Pro96).

Description of Embodiments

In the present invention and the present specification, the term "antibody light chain" refers to an immunoglobulin light chain. The animal species serving as the origin of the antibody light chain is not particularly limited, but it is preferably derived from a mammal, more preferably from a human or a mouse.

In the present invention and the present specification, the term "anticancer agent" means a drug having an activity of killing cancer cells or suppressing or inhibiting the proliferation thereof.

It is well known in the art that some amino acids of an amino acid residue constituting a polypeptide can be readily modified without significantly affecting the structure or function of the polypeptide. Furthermore, it is also well known that not only artificially modified proteins but also naturally occurring proteins have variants that do not significantly change the structures or functions of the proteins. In the present specification, substituting, adding, or deleting one or more amino acids in a specific amino acid sequence X is referred to as mutating.

Numbering of amino acid sequences in accordance with the Kabat numbering scheme can be carried out, for example, by analyzing amino acid sequences of an antibody in accordance with the Kabat numbering system with the abYsis software (URL: http://www.abysis.org/) or Discovery Studio (Accelrys Software, San Diego, CA, USA).

An aspect of the polypeptide derived from an anti-HER2 antibody light chain according to the present invention is a polypeptide derived from an anti-HER2 antibody light chain in which the hypervariable region is selected from the group consisting of (1a) to (1c):

(1a) a polypeptide consisting of an amino acid sequence that includes a CDR1 region consisting of an amino acid sequence set forth in SEQ ID NO: **1,** a CDR2 region consisting of an amino acid sequence set forth in SEQ ID NO: 2, and a CDR3 region consisting of an amino acid sequence set forth in SEQ ID NO: 3, and has an aspartic acid residue at position 70 of a variable region in accordance with the Kabat numbering scheme, wherein proline residues at positions 95 and 96 of the variable region in accordance with the Kabat numbering scheme are deleted or substituted with amino acid residues other than proline residues;

(1b) a polypeptide consisting of an amino acid sequence that includes a CDR1 region consisting of an amino acid sequence in which one or several amino acids are substituted, added, or deleted in the amino acid sequence set forth in SEQ ID NO: 1, a CDR2 region consisting of an amino acid sequence in which one or several amino acids are substituted, added, or deleted in the amino acid sequence set forth in SEQ ID NO: 2, and a CDR3 region consisting of an amino acid sequence set forth in SEQ ID NO: 3, and has an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues; and

(1c) a polypeptide consisting of an amino acid sequence that includes a CDR1 region consisting of an amino acid sequence having 90% or more identity with the amino acid sequence set forth in SEQ ID NO: 1, a CDR2 region consisting of an amino acid sequence having 90% or more identity with the amino acid sequence set forth in SEQ ID NO: 2, and a CDR3 region consisting of an amino acid sequence set forth in SEQ ID NO: 3, and has an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues.

The variable region of the light chain (VL region) consists of three hypervariable regions (CDRs: CDR1, CDR2, and CDR3) and four framework regions (FRs) surrounding the three CDRs, although the CDRs include specific sequences in this aspect.

The polypeptide (1a) consists of an amino acid sequence that includes a CDR1 region consisting of an amino acid sequence set forth in SEQ ID NO: 1, a CDR2 region consisting of an amino acid sequence set forth in SEQ ID NO: 2, and a CDR3 region consisting of an amino acid sequence set forth in SEQ ID NO: 3, and has an aspartic acid residue at position 70 of a variable region in accordance with the Kabat numbering scheme, wherein proline residues at positions 95 and 96 of a variable region in accordance with the Kabat numbering scheme are deleted or substituted with amino acid residues other than proline residues. Here, examples of the amino acid residues other than proline residues include lysine, arginine, histidine, aspartic acid, glutamic acid, serine, threonine, asparagine, glutamine, alanine, glycine, valine, isoleucine, leucine, methionine, and cysteine, among which serine or arginine is preferred. It is more preferable that prolines at positions 95 and 96 be deleted.

The polypeptide (1b) consists of an amino acid sequence in which the CDR regions include a CDR1 region consisting of an amino acid sequence in which one or several amino acids are substituted, added, or deleted in the amino acid sequence set forth in SEQ ID NO: 1, a CDR2 region consisting of an amino acid sequence in which one or several amino acids are substituted, added, or deleted in the amino acid sequence set forth in SEQ ID NO: 2, and a CDR3 region consisting of an amino acid sequence set forth in SEQ ID NO: 3, the amino acid sequence having an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues. Here, examples of the amino acid residues other than proline residues include lysine, arginine, histidine, aspartic acid, glutamic acid, serine, threonine, asparagine, glutamine, alanine, glycine, valine, isoleucine, leucine, methionine, and cysteine, among which serine or arginine is preferred. It is more preferable that prolines at positions 95 and 96 be deleted.

For example, substitution, addition, or deletion of one or several amino acids in the amino acid sequence set forth in SEQ ID NO: 1, 2, or 3 is preferably substitution, addition, or deletion of one or two amino acids, more preferably substitution, addition, or deletion of one amino acid. Here, examples of an amino acid substitution that may generally occur include alanine/serine, valine/isoleucine, aspartic acid/glutamic acid, threonine/serine, alanine/glycine, alanine/threonine, serine/asparagine, alanine/valine, serine/glycine, tyrosine/phenylalanine, alanine/proline, lysine/arginine, aspartic acid/asparagine, leucine/isoleucine, leucine/valine, alanine/glutamic acid, and aspartic acid/glycine.

The polypeptide (1c) consists of an amino acid sequence in which the CDR regions include a CDR1 region consisting of an amino acid sequence having 90% or more identity with the amino acid sequence set forth in SEQ ID NO: 1, a CDR2 region consisting of an amino acid sequence having 90% or more identity with the amino acid sequence set forth in SEQ ID NO: 2, and a CDR3 region consisting of an amino acid sequence set forth in SEQ ID NO: 3, and has an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues. Here, examples of the amino acid residues other than proline residues include lysine, arginine, histidine, aspartic acid, glutamic acid, serine, threonine, asparagine, glutamine, alanine, glycine, valine, isoleucine, leucine, methionine, and cysteine, among which serine or arginine is preferred. It is more preferable that prolines at positions 95 and 96 be deleted.

For example, the amino acid sequence having 90% or more identity with the amino acid sequence set forth in SEQ ID NO: 1, 2, or 3 preferably has 95% or more identity, more preferably 97% or more identity, even more preferably 98% or more identity, and still more preferably 99% or more identity.

The sequence identity of a target amino acid sequence with respect to the reference amino acid sequence can be determined, for example, as follows. First, the reference amino acid sequence and the target amino acid sequence are aligned. Here, the respective amino acid sequences may include gaps to maximize the sequence identity. Subsequently, the number of coincident amino acids is calculated for the reference amino acid sequence and the target amino acid sequence, and the sequence identity can be determined according to the following equation.

"Sequence identity (%)" = [Number of coincident amino acids]/[total number of amino acids of target amino acid sequence] $\times$ 100

Of the polypeptides (1a), (1b), and (1c), the polypeptide (1a) is more preferable.

An even more preferable polypeptide (1a) comprises a CDR1 region consisting of the amino acid sequence set forth in SEQ ID NO: 1, a CDR2 region consisting of the amino acid sequence set forth in SEQ ID NO: 2, and a CDR3 region consisting of SEQ ID NO: 5, and has an aspartic acid residue at position 70 of the variable region in accordance with the Kabat numbering scheme.

Another aspect of the polypeptide derived from an anti-HER2 antibody light chain according to the present invention is a polypeptide derived from an anti-HER2 antibody light chain in which the variable region is selected from the group consisting of (2a) to (2c):

(2a) a polypeptide consisting of an amino acid sequence set forth in SEQ ID NO: 4;

(2b) a polypeptide consisting of an amino acid sequence in which one or several amino acids are substituted, added, or deleted in the amino acid sequence set forth in SEQ ID NO: 4, the amino acid sequence having an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues; and

(2c) a polypeptide consisting of an amino acid sequence having 90% or more identity with the amino acid sequence set forth in SEQ ID NO: 4, the amino acid sequence having an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues.

In this aspect, the entire variable region (including CDRs and frameworks) of the polypeptide derived from the anti-HER2 antibody light chain according to the present invention includes a specific sequence. The substitution, addition, and deletion in the polypeptides of (2a), (2b), and (2c); the identity of the amino acid sequence; and the deletion of an amino acid residue or the substitution of the amino acid residue with an amino acid residue other than proline are the same as in the case if the polypeptides of (1a), (1b), and (1c).

Of the polypeptides (2a), (2b), and (2c), the polypeptide (2a) is more preferable. An even more preferable polypeptide (2a) consists of an amino acid sequence set forth in SEQ ID NO: 6.

Since the polypeptide derived from the anti-HER2 antibody light chain according to the present invention has the amino acid sequence as described above, it can be produced by various methods. However, the polypeptide is preferably produced based on the following two aspects, including a modification step and an expression step.

Another aspect of the present invention is a method for producing a polypeptide derived from an anti-HER2 antibody light chain having enzymatic activity or improved enzymatic activity, the method comprising:

a modification step of modifying a polynucleotide encoding an anti-HER2 antibody light chain in which a hypervariable region is (1a) a polypeptide comprising a CDR1 region consisting of an amino acid sequence set forth in SEQ ID NO: 1, a CDR2 region consisting of an amino acid sequence set forth in SEQ ID NO: 2, and a CDR3 region consisting of an amino acid sequence set forth in SEQ ID NO: 3; (1b) a polypeptide comprising a CDR1 region consisting of an amino acid sequence in which one or several amino acids are substituted, added, or deleted in the amino acid sequence set forth in SEQ ID NO: 1, a CDR2 region consisting of an amino acid sequence in which one or several amino acids are substituted, added, or deleted in the amino acid sequence set forth in SEQ ID NO: 2, and a CDR3 region consisting of an amino acid sequence set forth in SEQ ID NO: 3; or (1c) a polypeptide comprising a CDR1 region consisting of an amino acid sequence having 90% or more identity with the amino acid sequence set forth in SEQ ID NO: 1, a CDR2 region consisting of an amino acid sequence having 90% or more identity with the amino acid sequence set forth in SEQ ID NO: 2, and a CDR3 region consisting of an amino acid sequence set forth in SEQ ID NO: 3, wherein the polypeptide consists of an amino acid sequence in which amino acid residues at positions 95 and 96 of the variable region in accordance with the Kabat numbering scheme are proline residues, so that the proline residues are deleted or substituted to give a polynucleotide encoding a polypeptide derived from an anti-HER2 antibody light chain consisting of an amino acid sequence in which amino acid residues at positions 95 and 96 of the variable region in accordance with the Kabat numbering scheme are deleted or substituted with amino acid residues other than proline residues; and

an expression step of expressing a polypeptide derived from an anti-HER2 antibody light chain having enzymatic activity, by using an expression vector comprising a polynucleotide encoding an anti-HER2 antibody light chain after the modification step, in an intracellular or extracellular expression system.

Further, another aspect of the present invention is a method for producing a polypeptide derived from an anti-HER2 antibody light chain having enzymatic activity or improved enzymatic activity, the method comprising:

a modification step of modifying a polynucleotide encoding a polypeptide derived from an anti-HER2 antibody light chain in which a variable region is (2a) a polypeptide consisting of an amino acid sequence set forth in SEQ ID NO: 4; (2b) a polypeptide consisting of an amino acid sequence in which one or several amino acids are substituted, added, or deleted in the amino acid sequence set forth in SEQ ID NO: 4, the amino acid sequence having an aspartic acid residue at position 70 of a variable region in accordance with the Kabat numbering scheme; or (2c) a polypeptide having 90% or more identity with the amino acid sequence set forth in SEQ ID NO: 4 and having an aspartic acid residue at position 70 of a variable region in accordance with the Kabat numbering scheme, wherein the polypeptide consists of an amino acid sequence in which amino acid residues at positions 95 and 96 of the variable region in accordance with the Kabat numbering scheme are proline residues, so that the proline residues are deleted or substituted to give a polynucleotide encoding a polypeptide derived from an anti-HER2 antibody light chain, wherein the variable region is a polypeptide consisting of an amino acid sequence in which amino acid residues at positions 95 and 96 of the variable region in accordance with the Kabat numbering scheme are deleted or substituted with amino acid residues other than proline residues; and

an expression step of expressing a polypeptide derived from an anti-HER2 antibody light chain having enzymatic activity, by using an expression vector comprising a polynucleotide encoding an anti-HER2 antibody light chain after the modification step, in an intracellular or extracellular expression system.

In the modification step, a polynucleotide encoding an antibody light chain whose variable region is the polypeptide consisting of an amino acid sequence in which amino acid residues at positions 95 and 96 of the variable region in accordance with the Kabat numbering scheme are proline residues is modified so that the proline residues are deleted or substituted to give a polynucleotide encoding an antibody light chain whose variable region is a polypeptide consisting of an amino acid sequence in which amino acid residues at positions 95 and 96 of the variable region in accordance with the Kabat numbering scheme are deleted or substituted with amino acid residues other than proline residues.

This step can be performed to impart enzymatic activity to an antibody light chain that lacks enzymatic activity, or to improve the enzymatic activity of an antibody light chain that exhibits only low activity. That is, this step can also be referred to as a "method of imparting enzymatic activity to an antibody light chain that lacks enzymatic activity" or a "method of improving enzymatic activity of an antibody light chain".

As used herein, improving enzymatic activity of an antibody light chain means that the enzymatic activity of the antibody light chain after modification is increased by, for example, by 10%, 20%, 30%, 40%, 50%, 60%, 70%, 80%, 90%, 100%, 200%, 300% or more, compared to the enzymatic activity of the antibody light chain before modification.

A method of modifying the proline residues to be deleted or substituted may be performed using a well-known technique to readily mutate proline residues present at positions 95 and 96 in the amino acid sequence of the variable region in accordance with the Kabat numbering scheme among the amino acid residues constituting the polypeptide. For example, it is possible to mutate any one of bases of a polynucleotide encoding a polypeptide with the use of a known point mutagenesis. It is also possible to design a primer corresponding to any part of the polynucleotide encoding a polypeptide to fabricate a deletion variant or an insertion variant.

In the expression step, an antibody light chain is expressed in an expression system known in the art, such as an intracellular or extracellular expression system, by using an expression vector comprising a polynucleotide encoding an antibody light chain after the modification step. This expression step is also appropriately used as a method for producing a HER2-binding polypeptide comprising the polypeptide of the present invention derived from an anti-HER2 antibody light chain having HER2 decomposition activity or a HER2-binding antibody comprising the polypeptide of the present invention derived from an anti-HER2 antibody light chain having HER2 decomposition activity, which will be described later, independently from the modification step.

In the case of using a recombinant expression system, it is possible to employ a method of purifying a polypeptide formed by incorporating a polynucleotide encoding the antibody light chain according to the present invention into a recombinant expression vector, followed by introduction into a host capable of being expressed by a known manner, and then performing translation in the host (transformant). The recombinant expression vector may or may not be a plasmid, and is only required to enable the target polynucleotide to be introduced into the host.

In the case of introducing an exogenous polynucleotide into a host in this manner, a promoter that functions in the host so as to express the exogenous polynucleotide is preferably incorporated into the expression vector. Although the method of purifying the recombinantly produced polypeptide varies depending on the properties of the host and polypeptide used, a target polypeptide can be purified comparatively easily using a tag and the like.

In the case of using a cell-free expression system (cell-free protein synthesis system), a polynucleotide encoding the antibody light chain according to the present invention is preferably added to a solution containing components such as ribosomes or t-RNA required for protein translation and synthesis, followed by incubation at a suitable temperature, to purify the synthesized polypeptide.

Examples of the cell-free protein synthesis system include a system using a wheat germ extract, a system using a rabbit reticulocyte extract, a system using an *Escherichia coli* S30 extract, and a system using a cell component extract that can be obtained from devacuolated protoplast of plants. Generally, in order to translate eukaryote genes, a eukaryotic cell system, that is, the system using a wheat germ extract or the system using a rabbit reticulocyte extract, is selected. However, in view of the origins (prokaryote/eukaryote) of the genes to be translated or the application of the protein after the synthesis, any one of the synthetic systems can be employed. Various commercially available kits can be used for these synthesis systems.

Furthermore, since various viral gene products often express activity through a complex biochemical reaction involving the cytomembrane, such as the endoplasmic reticulum or Golgi bodies, following translation, it is necessary to add cytomembrane components (such as microsomal membrane) in order to reproduce the various biochemical reactions in vitro. Cell component extracts obtained from plant devacuolated protoplasts are preferable since they can be used as a cell-free protein synthesis liquid that retains cytomembrane components, thereby eliminating the need to add microsomal membrane.

As used herein, "cytomembrane components" are intended to refer to cell organelles composed of lipid membrane present in the cytoplasm (i.e., all types of intracellular granules such as endoplasmic reticulum, Golgi bodies, mitochondria, chloroplasts and vacuoles). In particular, since endoplasmic reticulum and Golgi bodies fulfill an important role in post-translation modification of proteins, they are essential cell components for maturation of membrane proteins and secretory proteins.

The polypeptide derived from the antibody light chain according to the present invention can also be obtained from cells or tissues that express the antibody light chain in nature. For example, cells or tissues that express the antibody light chain according to the present invention in nature can be identified using an antibody or an oligonucleotide.

In addition, the polypeptide derived from the antibody light chain according to the present invention can also be chemically synthesized. The chemical synthesis method is not particularly limited, and any of the methods used for chemically synthesizing a polypeptide may be adopted.

The polypeptide derived from the antibody light chain of the present invention obtained in the expression step can be purified using a known antibody purification method, but a purification method including a first purification step, an addition step, and a second purification step as described in Patent Literature 5 can also be used.

The polypeptide derived from the anti-HER2 antibody light chain of the present invention thus obtained has a HER2 decomposition enzymatic activity close to 4 times that of the antibody light chain before modification, as described in Examples below. A component selected from the group consisting of the polypeptide derived from the anti-HER2 antibody light chain, a HER2-binding polypeptide comprising the polypeptide derived from the light chain, and a functional fragment of the HER2-binding polypeptide comprising the polypeptide derived from the light chain is useful as an anticancer agent targeting HER2, the anticancer agent having, in addition to HER2 decomposition activity, one or more activities selected from the group consisting of antibody-dependent cellular cytotoxicity, complement-dependent cytotoxicity, and neutralizing activity.

As a part of factorial analysis of the enzymatic activity of the polypeptide derived from the light chain of the anti-HER2 antibody of the present invention, computer simulation of the conformation of the antibody light chain was performed. It seems that S26/R24/D70 is a suitable conformation for HER2 hydrolysis instead of the conventional structure (D1/S27a/H93) of the catalytic triad residues. Although not intending to be bound by a particular theory, the polypeptide derived from the anti-HER2 antibody light chain of the present invention is supposed to express high enzymatic activity due to the structure of the catalytic triad residues of S26/R24/D70.

Another aspect of the present invention is an anticancer agent comprising a component selected from the group consisting of: the polypeptide derived from an anti-HER2 antibody light chain of the present invention; a HER2-binding polypeptide comprising the polypeptide derived from the light chain; and a functional fragment of the HER2-binding polypeptide comprising the polypeptide derived from the light chain.

Another aspect of the present invention is a pharmaceutical composition for treating cancer, comprising a component selected from the group consisting of: the polypeptide derived from an anti-HER2 antibody light chain of the present invention; a HER2-binding polypeptide comprising the polypeptide derived from the light chain; and a functional fragment of the HER2-binding polypeptide comprising the polypeptide derived from the light chain.

Another aspect of the present invention is use of a component for production of an anticancer agent, the component being selected from the group consisting of: the polypeptide derived from an anti-HER2 antibody light chain of the present invention; a HER2-binding polypeptide comprising the polypeptide derived from the light chain; and a functional fragment of the HER2-binding polypeptide comprising the polypeptide derived from the light chain.

Another aspect of the present invention is a component for use in treatment of cancer, the component being selected from the group consisting of: the polypeptide derived from an anti-HER2 antibody light chain of the present invention; a HER2-binding polypeptide comprising the polypeptide derived from the light chain; and a functional fragment of the HER2-binding polypeptide comprising the polypeptide derived from the light chain.

Another aspect of the present invention is a method for treating cancer, the method comprising administering a component selected from the group consisting of: the polypeptide derived from an anti-HER2 antibody light chain of the present invention; a HER2-binding polypeptide comprising the polypeptide derived from the light chain; and a functional fragment of the HER2-binding polypeptide comprising the polypeptide derived from the light chain.

The active ingredient used in the anticancer agent includes one or more components selected from the group consisting of: the polypeptide derived from an anti-HER2 antibody light chain of the present invention; a HER2-binding polypeptide comprising the polypeptide derived from the light chain; and a functional fragment of the HER2-binding polypeptide comprising the polypeptide derived from the light chain.

Examples of the polypeptide derived from an anti-HER2 antibody light chain of the present invention includes a polypeptide selected from the group consisting of the above mentioned (1a), (1b), and (1c), a polypeptide selected from the group consisting of the above mentioned (2a), (2b), and (2c), and a whole light chain including these polypeptides and a light-chain constant region.

The HER2-binding polypeptide comprising a light chain may be an antibody comprising a constant region, or an Fc region. The class of the antibody is not particularly limited, and antibodies having any isotypes, such as IgG, IgM, IgA, or IgD are included. In consideration of, for example, the ease of purification, IgG is preferred.

The HER2-binding polypeptide comprising a light chain also include a monoclonal antibody, a polyclonal antibody, and a HER2-binding antibody comprising the light chain, such as an antibody that retain the ability to specifically bind to an antigenic determinant, as well as a variant and derivative of an antibody, such as a T-cell receptor fragment.

In addition, the type of the HER2-binding antibody comprising a light chain is not particularly limited, and mouse antibodies, human antibodies, rat antibodies, rabbit antibodies, sheep antibodies, camel antibodies, avian antibodies, or the like, and recombinant antibodies artificially modified for the purpose of reducing heterologous antigenicity against humans, for example, chimeric antibodies, humanized antibodies, and the like can be appropriately used.

The functional fragment of the HER2-binding polypeptide including the polypeptide derived from the light chain may be a low molecular weight antibody such as an antibody fragment (fragment), a modified product of an antibody, and the like. Specific examples of the antibody fragment include Fv, Fab, F(ab')$_2$, Fab-Fc, F(ab')$_2$-pFc'scFab, and scFv, where the scFv includes tandem di-scFv, diabody, tandem tri-scFv, and tri(a)body. More specifically, VL-CL, VL-VH, VL-VL, VL-VL-VL, and the like are included, and in view of improving the valence of the light chain of the anti-HER2 antibody of the present invention, scFv such as VL-VL and VL-VL-VL are preferred.

Next, specific examples of the HER2-binding polypeptide including the polypeptide derived from the light chain, the HER2-binding antibody comprising the polypeptide derived from the light chain, and the functional fragment of the HER2-binding polypeptide the polypeptide derived from the light chain include:

(a) a polypeptide comprising a variable region of an anti-HER2 antibody heavy chain as set forth in SEQ ID NO: 12 in addition to a polypeptide derived from an anti-HER2 antibody light chain of the present invention (a polypeptide selected from the group consisting of the above mentioned (1a), (1b) and (1c), or a polypeptide selected from the group consisting of the above mentioned (2a), (2b) and (2c));

(b) a polypeptide comprising a variable region of an anti-HER2 antibody heavy chain as set forth in SEQ ID NO: 12 linked to a polypeptide derived from an anti-HER2 antibody light chain of the present invention (a polypeptide selected from the group consisting of (1a), (1b), and (1c), or a polypeptide selected from the group consisting of (2a), (2b), and (2c)) via a linker;

(c) a polypeptide in which two polypeptides (b) are linked via a linker;

(d) a polypeptide comprising a constant region of a light chain linked to a polypeptide derived from an anti-HER2 antibody light chain of the present invention (a polypeptide selected from the group consisting of (1a), (1b), and (1c), or a polypeptide selected from the group consisting of (2a), (2b), and (2c)), and a heavy chain CH1 region linked to a variable region of an anti-HER2 antibody heavy chain as set forth in SEQ ID NO: 12;

(e) the polypeptide (d) in which the variable region of the light chain and the variable region of the heavy chain are linked via a linker;

(f) a polypeptide further comprising an Fc region linked to the heavy chain CH1 region of the above mentioned (d) or (e) above;

the polypeptide (c), in which a heavy chain CH1 region linked to a heavy-chain variable region set forth in SEQ ID NO: 12 linked to a polypeptide derived from an anti-HER2 antibody light chain of the present invention (a polypeptide selected from the group consisting of (1a), (1b), and (1c), or a polypeptide selected from the group consisting of (2a), (2b), and (2c)), or the whole light chain comprising these polypeptides and a constant region of the light chain) is associated via a disulfide bond; and

(g) a polypeptide comprising a heavy-chain CH2 and a heavy-chain CH3 that are linked to a CH1 region of the above mentioned polypeptide (f).

The two or more polypeptides are preferably linked by a covalent bond, such as a polypeptide bond. The covalent bond can be a direct polypeptide bond or an indirect polypeptide bond through a linker sequence, for which flexible amino acids, selected from, for example, glycine, alanine, and serine, are usually used. A linker with greater rigidity can also be used. The length of the linker sequence can be readily determined.

The linker sequence is often about 12 amino acids or less in length, for example 10 amino acids or less or 2 to 10 amino acids in length, and examples of non-covalent bonds include hydrogen bonds, van der Waals forces, and ionic bonds.

The term "associate(d)" refers to a bond in quaternary structure between polypeptides, that is, a bond between polypeptides having a tertiary structure in a case where a plurality of polypeptides having a tertiary structure associate with each other to form a quaternary structure and form a single molecule. In one aspect of the present invention, a polypeptide comprising a light-chain variable region and a light-chain constant region is associated with a polypeptide comprising a heavy-chain variable region and a heavy-chain constant region via disulfide bonds, hydrogen bonds, van der Waals forces, ionic bonds, and the like. The above mentioned polypeptide (g) can be obtained by expressing it using an expression vector comprising a polynucleotide encoding a polypeptide in which the polypeptide of the present invention consisting of the amino acid sequence set forth in SEQ ID NO: 6 and the polypeptide set forth in SEQ ID NO: 15 are linked to each other, by an expression system known in the art such as an intracellular or extracellular expression system. In an intracellular expression system, for example, Chinese hamster ovary cells (CHO cells) may be used as a host cell.

An antibody bound to various molecules such as polyethylene glycol (PEG) can also be used as an antibody modifier. Such an antibody modifier can be obtained by chemically modifying an obtained antibody.

In the present invention, it is also possible to use an antibody with a modified sugar chain or the like, for the purpose of enhancing cytotoxicity, or the like. Furthermore, in the present invention, HEXABODY technology or the like, which imparts complement-dependent cytotoxicity, can also be appropriately applied.

The anticancer agent of the present invention is effective against cancers that excessively express HER2, and is preferably used for the treatment of breast cancer, gastric cancer, salivary gland cancer, and other cancers that excessively express HER2, for example.

The anticancer agent of the present invention may be administered orally or parenterally to animals including humans having cancer, and the parenteral administration is preferable. Specific administration methods include mucosal administration, intramuscular administration, intravenous administration, subcutaneous administration, ocular administration or transdermal administration. The anticancer agent of the present invention can be administered at a dose of preferably 0.01 to 30 mg/kg body weight, more preferably 0.1 to 10 mg/kg body weight, and even more preferably 0.1 to 1 mg/kg body weight as the protein mass of the active ingredient. As an administration method, after the agent is administered once, it can also be administered at an interval of a certain period, for example, 2 to 22 days.

The anticancer agent of the present invention can be made into an anticancer agent composition containing, in addition to the aforementioned active ingredients, a pharmaceutically acceptable carrier, a diluent or an excipient (including combinations thereof). The pharmaceutically acceptable carrier or excipient is well known in the pharmaceutical art and is described, for example, in Remington's Pharmaceutical Sciences, Mack Publishing Co. (A. R. Gennaro, Eds., 1985). A pharmaceutically usable carrier, excipient, or diluent can be readily selected by those skilled in the art, in accordance with the routes of administration and standard pharmaceutical practice. The anticancer agent composition of the present invention may also contain any suitable binders, lubricants, suspending agents, coating agents or solubilizing agents.

Example

Next, the present invention will be described in more detail with reference to examples, but the present invention is not limited to these examples.

Example 1 (Production and Enzymatic Activity of Antibody Light Chain-Derived Polypeptide of the Present Invention)

a. Mutagenesis in Antibody Light Chain

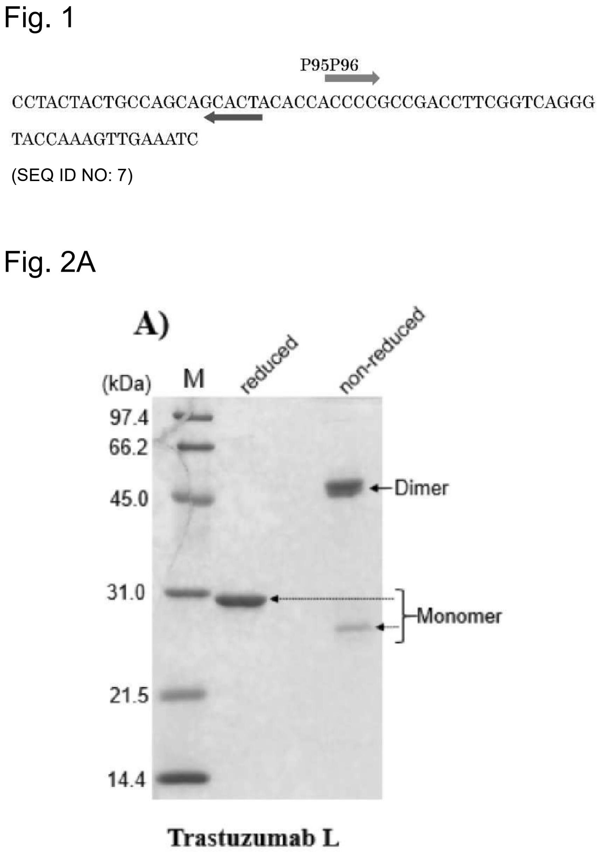

(1) The sequence around P95P96 of the trastuzumab light chain is illustrated in Figure 1 and set forth in SEQ ID NO: 7.
(2) Primer Sequences and Template DNA

Forward primer (red): ACC TTC GGT CAG GGT ACC AAA GTT GA (SEQ ID NO: 8)
Reverse primer (blue): GGT GGT GTA GTG CTG CTG GCA GTA G (SEQ ID NO: 9)
trastuzmab-L/pET20b(+) 113.5 ng/μL

(3) Procedure

A template plasmid was diluted to 50 ng/mL in sterilized MilliQ. All reagents except KOD-plus were added to a PCR tube, mixed, spun down, and collected at the tip of the tube.

[Table 1]

| Component | Final conc. | volume (μL) |
|---|---|---|
| 10×Buffer for iPCR | - | 5 |
| Sterilized MilliQ | - | 35 |
| 2 mM dNTPs | 200 mL | 5 |
| 10 pmol/mL Reverse primer | 0.3 pmol/ mL | 1.5 |
| 10 pmol/mL Forward primer | | 1.5 |
| 50 ng/mL plasmid | 1 ng/mL | 1 |

(continued)

| Component | Final conc. | volume (μL) |
|---|---|---|
| KOD-plus 1 U/mL | 0.02 U/mL | 1 |
| Total | - | 50 |

KOD-plus was added and gently tapped, followed by spin-down. Inverse PCR (iPCR) was performed using a thermal cycler.

Initial denaturation: 94°C, 2 min
Denaturation: 98°C, 10 sec
Annealing & Extension: 71°C, 5 min (five cycles), 4°C, Hold

A 10 μL portion of the iPCR reaction solution was frozen and stored for electrophoresis. To 40 μL of the remaining iPCR reaction solution was added 1.6 μL of Dpn I. After incubation at 37°C for one hour, the template DNA was digested. Agarose electrophoresis for confirmation of Dpn I digestion was performed.

For confirmation of band position, a template plasmid (100 ng/Lane) was used, and the iPCR solution was electrophoresed at 5 μL/Lane. The agarose concentration was 1%. Dpn I digestion could be confirmed. A self-ligation solution was prepared in the PCR tube according to the following table and reacted at 16°C for one hour.

[Table 2]

| Component | volume(μL) |
|---|---|
| Dpn I-treated PCR product | 2 |
| Sterilized MilliQ | 7 |
| Ligation high | 5 |
| T4 Polynucleotide Kinase | 1 |
| Total | 15 |

Competent Cell, DH5 was thawed in an ice-bath until it became sherbet-like, to which 10 μL of Ligation reaction solution was added and allowed to stand for 30 minutes. The mixture was immersed in a water bath at 42°C for 30 seconds, returned to the ice-bath, and allowed to stand for two minutes. To a round tube were added 900 μL of SOC medium and the Competent Cell, and a revival culture was performed at 37°C at 200 rpm for one hour. The revival culture solution was seeded on an LB plate containing 100 μg/mL of ampicillin (Amp) and cultured overnight at 37°C. The next day, 8 colonies were selected and subjected to streak culture using the same plate to prepare a Master plate.

Colony PCR was performed using bacterial cells from the Master plate. Plasmid was extracted from bacterial cells showing amplification around 660 bp (full-length light chain), and the sequence was confirmed by sequence analysis. Subsequently, glycerol stocks were prepared from two colonies, and the plasmid was simultaneously cloned into BL21 (DE3) pLysS to create bacterial cells for expression.

b. Culture, Recovery, and Purification

Transformants were grown in 1 L of Luria-Bertani medium containing 100 μg/mL ampicillin to A600 nm of 0.6 at 37°C and then incubated with 0.01 mM IPTG at 18°C for 20 hours. Cells were recovered by centrifugation (3500 g, 4°C, 10 min) and then resuspended in 100 mL of a solution of 250 mM NaCl, 25 mM Tris-HCl, pH 8.0. The cells were sonicated three times for two minutes each in an ice-bath and then centrifuged (21,475 g, 4°C, 20 min). An expressed human light chain was recovered as the supernatant.

The supernatant was first subjected to Ni-NTA column chromatography (Qiagen) equilibrated with 25 mM Tris-HCl, pH 8.0, containing 250 mM NaCl. Elution was performed by increasing the concentration of imidazole to 0 and/or 30 to 300 mM. After the Ni-NTA column chromatography was completed, a solution of 50 μM $CuCl_2$ (light chain: 1.25 eq) was added to the eluate, based on the calculation considering an absorbance of 1.0 at A600 nm in UV/VIS as about 1 mg/mL (40 μM light chain) (which is important to make a homogeneous (dimeric) structure). Then, the solution containing a light chain and copper ions were dialyzed against a 50 mM Tris-HCl buffer at pH 8.0 for about 20 hours. After removal of partial aggregates by centrifugation (17,800 g, 4°C, 20 min), the solution was concentrated to 2 mg/mL and subjected to cation exchange chromatography using a column of SP-5PW (Tosoh Corporation, Japan) with a gradient (from 0.0% to 15.0%) of NaCl in Tris-HCl (pH 8.0) buffer on a purification device (AKTA system, GE HealthCare Japan, Tokyo) or subjected to size exclusion chromatography on an AKTA system (column; HiLoad™ 16/60Superdex™ 200 pg (GE HealthCare) using a PBS (pH 7.4) buffer containing 137 mM NaCl as an eluent solvent, after which the eluent was collected and dialyzed against 20 mM Tris-HCl/150 mM NaCl buffer (pH 8.5) for about 17 hours. The solution was then concentrated using Amicon Ultra 10000 (Millipore, USA). EDTA was added to the solution so as to be a concentration of 50 mM, reacted for 1 hour at 4°C, and then dialyzed twice against 2 L of PBS. After confirming complete removal of Cu(II) by UV/VIS spectrophotometry, the solution was filtered through a 0.2 µm membrane filter (Merck-Millipore) and stored at 4°C or frozen. Protein concentration was determined using the DC Protein Assay Kit (Bio-Rad) by the Bradford method employing the Lowry procedure.

SDS-PAGE Analysis:

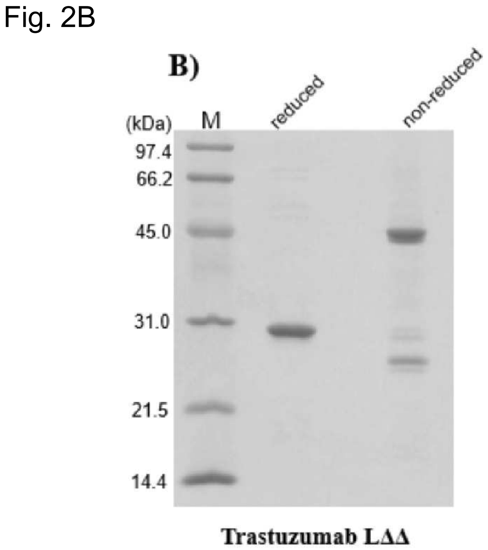

After the light chain purification, SDS-PAGE analysis with CBB staining was performed. The results for the trastuzumab light chain under reducing and non-reducing conditions are shown in Figure 2A. Here, approximately about 46 and 28 kD bands corresponding to the dimer and monomer, respectively, were seen under non-reducing conditions. Under reducing conditions, only the 28 kDa band corresponding to the monomer was detected. SDS-PAGE analysis showed few bands other than the monomer and dimer bands of the light chain, suggesting that the trastuzumab light chain was highly purified. Under non-reducing conditions, the monomer band was faint, but the dimer band was distinct, indicating that the dimer is dominant in solutions. Figure 2B shows the results of SDS-PAGE for variants of the light chain without Pro95 and Pro96. The results were similar to those obtained with the trastuzumab light chain, but under non-reducing conditions, a thin band was observed around 30 kDa.

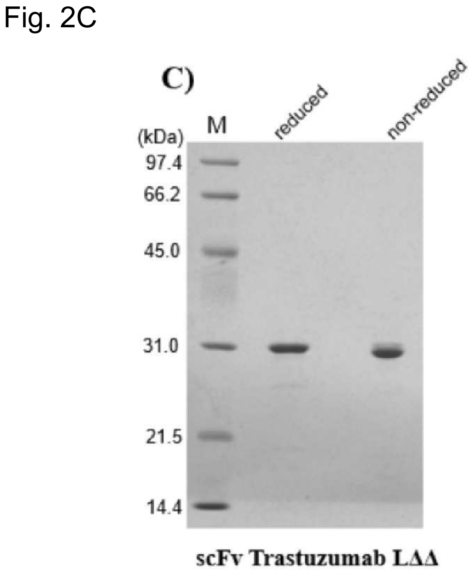

The results of SDS-PAGE of purified scFv trastuzumab light chain P95ΔP96Δ are illustrated in Figure 2C. In this case, a single band around 31 kDa was detected under both reducing and non-reducing conditions. No other bands were detected; thus, scFv trastuzumab light chain P95 Δ P96 Δ was highly purified.

c. Enzyme Activity Measurement Methods

(1) Method Using Arg-pNA Substrate

In 100 mM Glycine/50 mM Tris-HCl buffer containing 0.025% Tween 20 (TGT buffer; pH 7.7), the cleavage of the p-nitroanilide amide bond to the C-terminal aa in R-pNA substrate (Peptides Institute Inc., Osaka, Japan) was measured at 37°C in a 96-well plate (96-well plate/353075, Becton-Dickinson, New Jersey, USA). Purified light chain (20µL) was mixed with 180µL of the synthetic substrate R-pNA. The final concentrations of the light chain and the substrate were 10 µM and 200 µM, respectively. Para-nitroaniline formed from the substrate catalyzed by the light chain was detected by measuring absorbance at 405 nm, while 620 nm was used as a reference using a microplate reader (SkanIt 3.1 for the Multiskan FC, Thermo Fisher Scientific, Massachusetts, USA). The catalytic activity was estimated from the amount of p-nitroaniline produced.

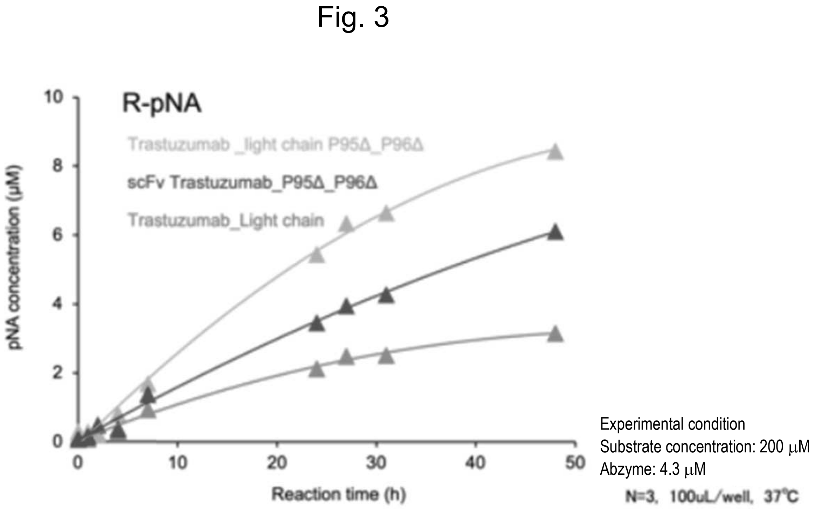

The trypsin-like substrate molecules, Arg-pNA substrate (hereafter, R-pNA), were used for investigation of the catalytic activities. The reaction time-course of R-pNA cleavage is shown in Figure 3. The three tested samples gradually hydrolyzed the synthetic substrate R-pNA, resulting in the formation of p-nitroaniline (pNA). The decomposition reactions were monitored up to 48 hours. Of the three samples, trastuzumab light chain, trastuzumab light chain P95ΔP96Δ, and ScFv trastuzumab light chain P95ΔP96Δ, trastuzumab light chain exhibited the lowest catalytic activities. Meanwhile, trastuzumab light chain P95ΔP96Δ exhibited the highest activities. The ScFv trastuzumab light chain P95ΔP96Δ exhibited an activity intermediate between those of trastuzumab light chain and trastuzumab P95ΔP96Δ. The initial reaction rates were estimated as 0.13 µM/hr for trastuzumab light chain, 0.24 µ M/hr for trastuzumab light chain P95 Δ P96Δ, and 0.20 µM/hr for ScFv trastuzumab light chain P95ΔP96Δ at a concentration of 4.3 µM of each of the molecules. Peptidase activity was similar to that of 22F6 human catalytic antibody (reaction rate: 0.09 µM/hr at 4.0 µM concentration of 22F6 catalytic antibody light chain).

(2) Method for Measuring Dissociation Constant (KD)

Binding affinity between HER2 and the trastuzumab light chain (Tmb-L-wt), trastuzumab light chain P95ΔP96Δ (Tmb-L-PPΔ), scFv trastuzumab light chain (Tmb-scFv(wt)), scFv trastuzumab light chain P95ΔP96Δ (Tmb-scFvPPΔ), and trastuzumab (Trastuzumab) were measured using a measuring device Octet N1 (Sartorius AG, Germany) and the Advanced Kinetics method, following the protocol. A recombinant HER2 molecule was bound to an Avidin-tag (SINO-Biochemical Laboratory; 10004-HCCH-B) using a streptavidin-immobilized biosensor chip (SA-biosensor). The experiment was performed as a five-step sequential assay at room temperature. First, the SA-biosensor was immersed for 30 seconds in 250 µL of PBS buffer in a microtube (ClickFit, LightBlock, PP, Black, 0.5 mL, TreffLab, Degersheim, Switzerland). Subsequently, the SA-biosensor was immersed in a Drop-Holder containing 4 µL of 100 nM recombinant HER2 molecules for 120 seconds. In the third step, the SA-biosensor (immobilized with a recombinant HER2 molecule) was immersed in PBS in a washing microtube. In the fourth step, the SA-biosensor bound to the recombinant HER2 molecule was immersed in a drop-holder containing 4 $\mu$L of antibody solution for 120 seconds. In the final (fifth) step, the SA-biosensor bound to the antibody was immersed again in the PBS buffer to measure dissociation. Data analysis was performed using Octet N1 software.

To obtain accurate assessments of the dissociation constant ($K_D$), association rate constant (ka), and dissociation rate constant (kd), various concentrations of each antibody were prepared. The dissociation constant ($K_D$) was calculated as kd/ka. As a result, as shown in Table 3, the dissociation constant ($K_D$) of trastuzumab light chain P95ΔP96Δ (Tmb-L-PPΔ) was extremely high, similar to that of trastuzumab light chain (Tmb-L-wt). Furthermore, the dissociation constant ($K_D$) of scFv trastuzumab light chain (Tmb-scFv(wt)) was nearly equivalent to that of scFv trastuzumab light chain P95ΔP96Δ (Tmb-scFvPPΔ).

| Antibodies | $K_D$ (nM) | $k_a=(M^{-1}s^{-1})$ | $k_d=(s^{-1})$ |
|---|---|---|---|
| Trastuzumab | 1.60 | 3.58 ± 0.80 (x10⁵) | 5.72 ± 0.3 9 (x10⁻⁴) |
| ScFv-Trastuzumab(wt) | 6.94 | 1.54 ± 0.13(x10⁵) | 10.7 ± 0.90(x10⁻³) |
| ScFv-Trastuzumab/PPΔ | 0.93 | 1.67 ± 0.03(x10⁵) | 1.55 ± 0.32(x10⁻⁴) |
| Trastuzumab-L-wt | 145 | 3.12 ± 0.09(x10⁴) | 4.52 ± 0.11(x10⁻³) |
| Trastuzumab-L-PPΔ | 123 | 2.09 ± 0.04(x10⁴) | 2.58 ± 0.05(x10⁻³) |

(3) Method Using FRET-HER2 Peptide (a)

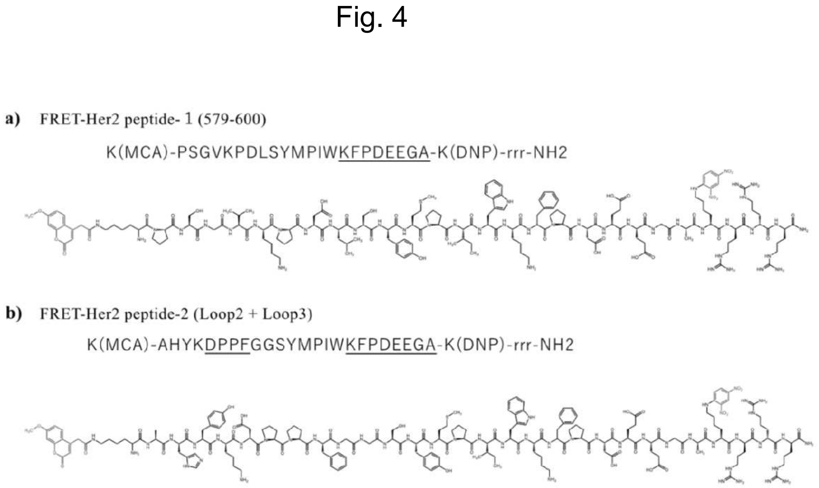

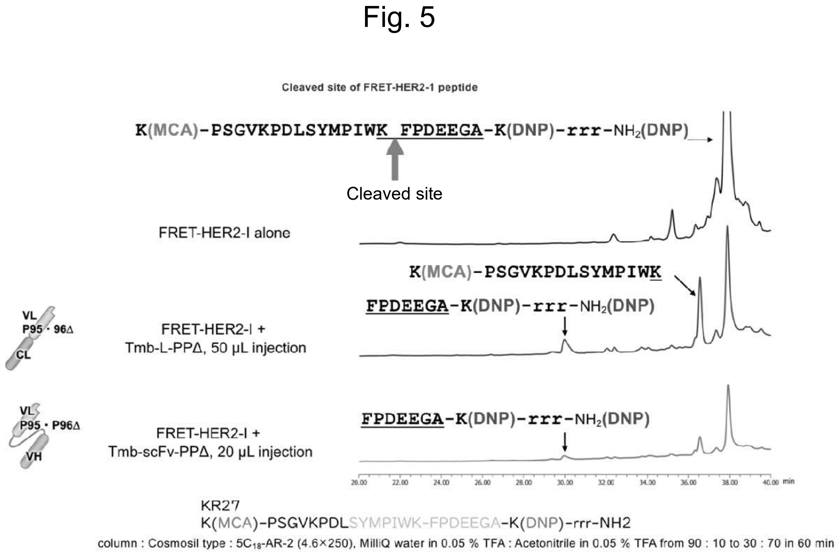

FRET-HER2 peptides were synthesized to measure catalytic activity and conduct kinetic studies. The targeting sequence of the Her2 molecule should include the epitope region of trastuzumab. The epitope sequence is considered to be aa563-626. The sequence Loop3 (PDB: 1N8Z, and 6OGE) [593]KFPDEEGA[600] strongly interacts with the light chain of trastuzumab (see PDB: 1N8Z and 6OGE). Therefore, the region of the following peptide [579]PSGVKPDLSYMPIWKFP-DEEGA[600] was extracted and synthesized as FRET-Her2 peptide-1 (a in Figure 4) (579-600). Figure 5 illustrates cleaved sites of the FRET-HER2 peptide-1 (579-600) and structures of decomposition products thereof.

FRET-HER2 peptide (25 $\mu$M) was incubated with trastuzumab light chain (Tmb-L), trastuzumab light chain P95ΔP96Δ (Tmb-L-PΔΔ), scFv trastuzumab light chain (Tmb-scFv), scFv trastuzumab light chain P95ΔP96Δ (Tmb-scFv), and trastuzumab (Trastuzumab) (5 $\mu$M) in a TGT buffer containing 0.02% NaN$_3$ at 37°C. Transmittance was measured periodically using Fluoroskan Ascent ($\lambda_{ex}$ = 320 nm, $\lambda_{em}$ = 405 nm; Thermo Fisher Scientific Oy, Vantaa, Finland). All measurements were performed twice.

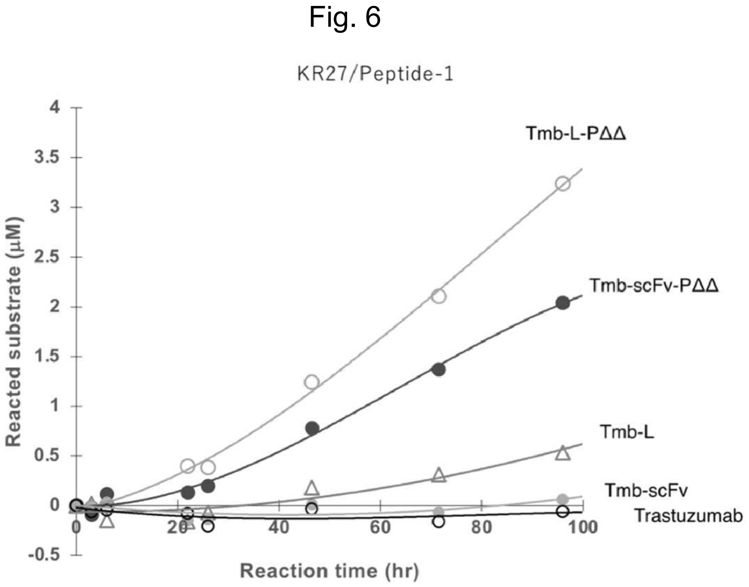

Figure 6 shows the results using FRET-HER2 peptide-1. In this case, the trastuzumab light chain P95ΔP96Δ exhibited the highest catalytic activity, and the scFv trastuzumab light chain P95ΔP96Δ exhibited the second highest catalytic activity. On the other hand, the trastuzumab light chain exhibited low activity, while the scFv trastuzumab light chain and trastuzumab exhibited the lowest activity. The order of the activity was similar to the results obtained in the Arg-pNA assay, with the trastuzumab light chain P95ΔP96Δ exhibiting the highest activity, followed by the scFv trastuzumab light chain P95ΔP96Δ.

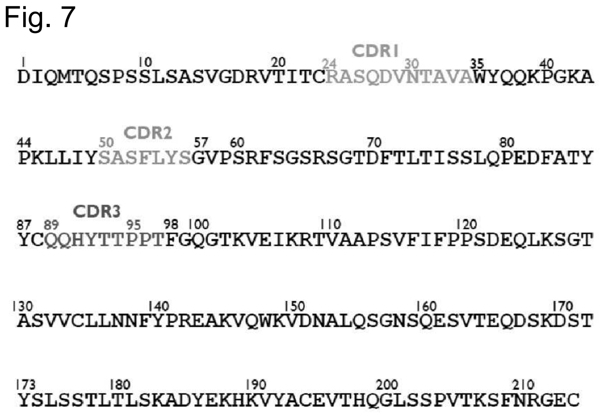

The amino acid sequence of a variable region of a trastuzumab light chain is illustrated in Figure 7 and set forth in SEQ ID NO: 4. The amino acid sequence of CDR1 of trastuzumab light chain is set forth in SEQ ID NO: 1, the amino acid sequence of CDR2 is set forth in SEQ ID NO: 2, and the amino acid sequence of CDR3 is set forth in SEQ ID NO: 3. The amino acid sequence of the polypeptide lacking P95 and P96 from the CDR3 region of the trastuzumab light chain is set forth in SEQ ID NO: 5.

The amino acid sequence of the polypeptide lacking P95 and P96 from the variable region of the trastuzumab light chain is set forth in SEQ ID NO: 6.

(4) Method Using FRET-HER2 Peptide (b)

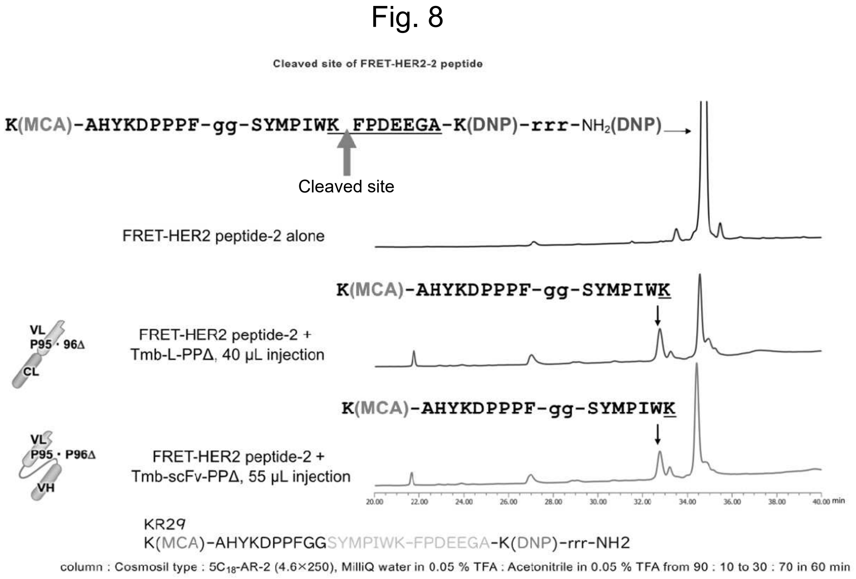

FRET-Her2 peptide-2 (Loop2 + Loop3) illustrated in Figure 4 b) was synthesized. Note that Figure 8 illustrates cleaved sites of the FRET-HER2 peptide-2 (Loop2 + Loop3) and structures of decomposition products thereof.

FRET-HER2 peptide-2 (25 $\mu$M) was incubated with trastuzumab light chain (Tmb-L), trastuzumab light chain P95ΔP96Δ (Tmb-L-PΔΔ), scFv trastuzumab light chain (Tmb-scFv), scFv trastuzumab light chain P95ΔP96Δ (Tmb-scFv), and trastuzumab (Trastuzumab) (5 $\mu$M) in a TGT buffer containing 0.02% NaN$_3$ at 37°C. Transmittance was measured periodically using Fluoroskan Ascent ($\lambda_{ex}$ = 320 nm, $\lambda_{em}$ = 405 nm; Thermo Fisher Scientific Oy, Vantaa, Finland). All measurements were performed twice.

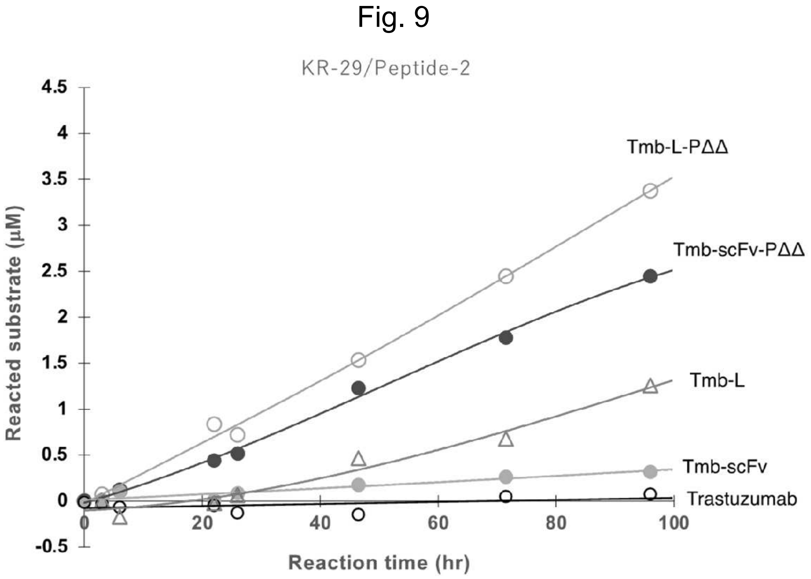

Figure 9 shows the results using FRET-HER2 peptide-2. In this case, the trastuzumab light chain P95ΔP96Δ exhibited the highest catalytic activity, and the scFv trastuzumab light chain P95ΔP96Δ exhibited the second highest catalytic activity. On the other hand, the trastuzumab light chain exhibited low activity, while the scFv trastuzumab light chain and trastuzumab exhibited the lowest activity. The order of the activity was similar to the results obtained in the Arg-pNA assay, with the trastuzumab light chain P95ΔP96Δ exhibiting the highest activity, followed by the scFv trastuzumab light chain P95ΔP96Δ.

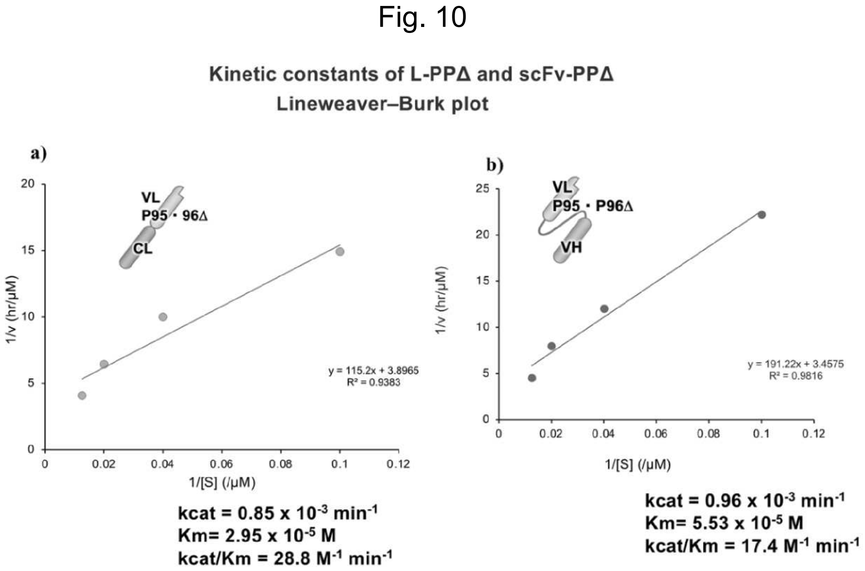

Figure 10 shows results of calculation of kinetic constants by Lineweaver-Burk plot for trastuzumab light chain P95ΔP96Δ and scFv trastuzumab light chain P95ΔP96Δ. Both showed high Km values.

Comparative Example 1 (Change in Enzymatic Activity Due to P95 Deletion in Antibody Light Chain Lacking D1/S27/H93)

P95 was deleted from the antibody light chain #4, which contained the amino acid sequence of SEQ ID NO: 10, in the same manner as in Example 1 to examine the change in the enzymatic activity.

As a result, no increase in enzymatic activity was observed for #4P95Δ.

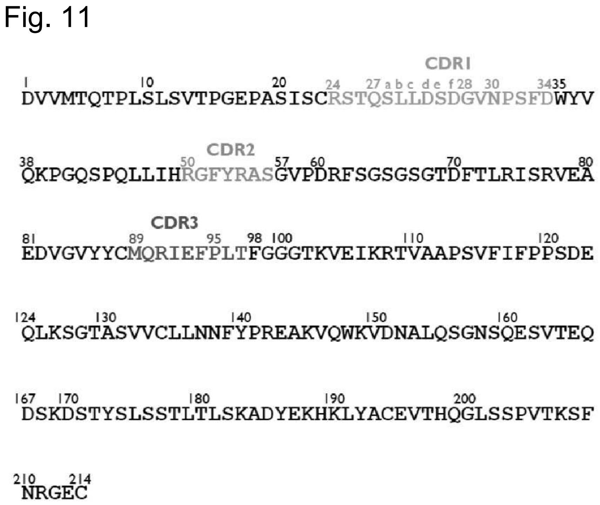

The amino acid sequence of #4 is illustrated in Figure 11 and set forth in SEQ ID NO: 10. The amino acid sequence of the polypeptide derived from #4 lacking P95 is set forth in SEQ ID NO: 11.

Example 2 (Conformation Simulation)

Conformation simulations were performed using molecular modeling of #4, #4P95Δ, trastuzumab light chain, trastuzumab light chain P95Δ, and trastuzumab light chain P95ΔP96Δ with Discovery Studio (Accelrys Software, San Diego, CA, USA).

As a result, #4 and #4P95Δ, D1/S27a/H49 were considered candidates for the catalytic triad residues, but construction of the catalytic triad residues was impossible.

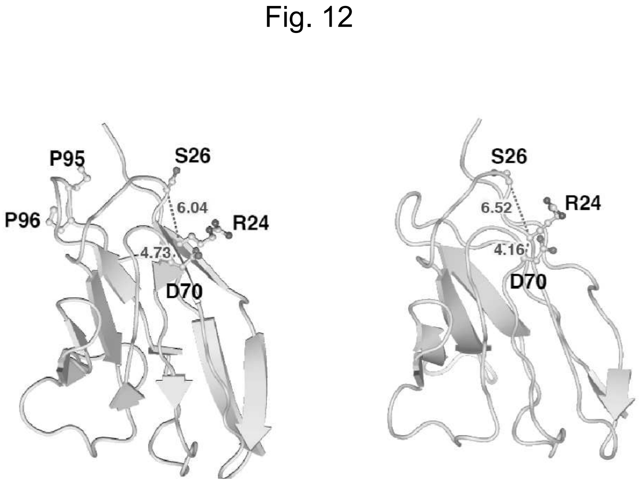

In the case of the trastuzumab light, D1/S26/H91 was considered candidates for the catalytic triad residues, but construction of the catalytic triad residues was impossible. On the other hand, quite unexpectedly, the catalytic triad residues of the trastuzumab light chain P95ΔP96Δ could be constructed by S26/R24/D70 (Figure 12, the left side is trastuzumab light chain and the right side is trastuzumab light chain P95ΔP96Δ in the figure). As a result, it was considered that the polypeptide derived from the anti-HER2 antibody light chain of the present invention exhibited enzymatic activity close to 4 times that of trastuzumab.

Example 3 (Cytotoxicity Assay of Polypeptide Derived from Anti-HER2 Antibody Light Chain of the Present Invention)

BT-474 clone 5 cells were cultured using 10% FBS-added RPMI-1640 medium (Sigma, R8005, high glucose) as the culture medium. Once the required number of cells for the logarithmic growth phase was reached, the cells were detached using 0.25% Trypsin-EDTA (Gibco, 25200-072) and adjusted to a concentration of $6.4 \times 10^4$ cells/mL in the culture medium. The cells were seeded at 100 μL/well into a 96-well plate and cultured at 5% $CO_2$, 37°C for 48 hours. The culture solution was removed, and 50 μL/well of RPMI-1640 medium was added. Subsequently, 50 μL/well of antibody solution adjusted to 7.06 μM in PBS was added. After gentle mixing, the cells were cultured at 37°C with 5% $CO_2$ for 72 hours, to which 2 μg/mL PI solution (Dojindo, 341-07881) and 2 μg/mL Hoechst 33342 solution (Dojindo, 346-07951) were added. The mixture was left to stand still at 37°C with 5% $CO_2$ for 30 minutes, followed by morphological observations using KEYENCE All-in-One Microscope (Model BZ-9000).

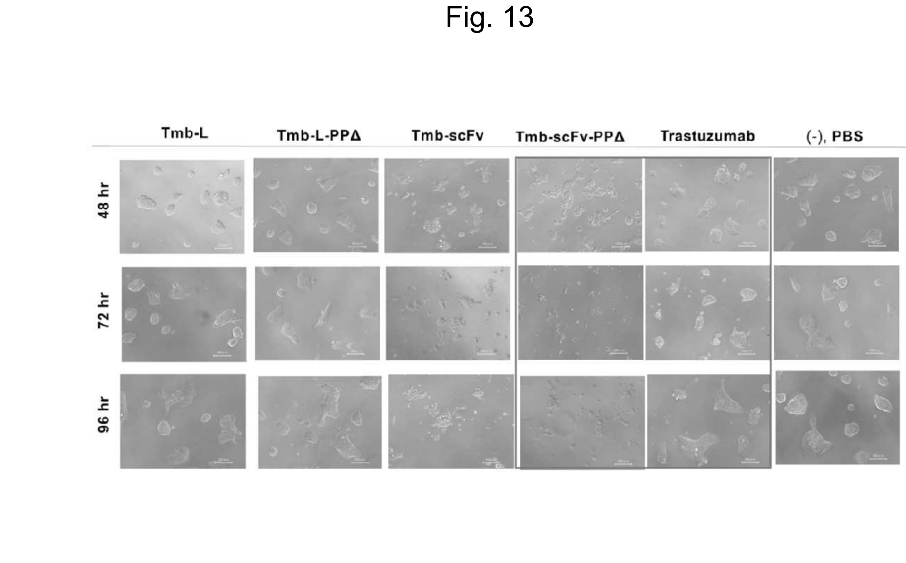

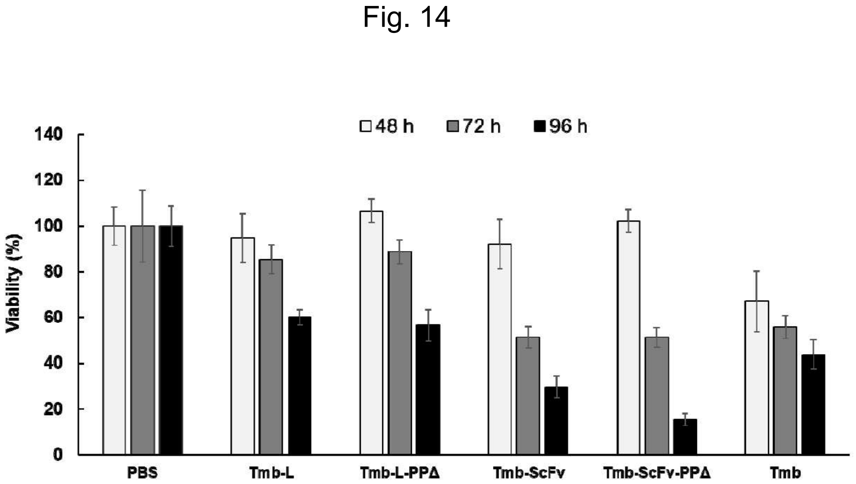

As a result, as shown in Figures 13 and 14, scFv trastuzumab light chain P95ΔP96Δ (Sc-FvP95Δ in the figure), trastuzumab light chain P95ΔP96Δ (L-P95Δ in the figure), and trastuzumab (Trasutuzumab in the figure) exhibited cytotoxicity against the human breast cancer cell line BT-474 in this order. The anti-human HER2 antibody light chain (Lchain in the figure) exhibited almost no cytotoxicity.

Example 4 (Relative Values in Antigen Decomposition Activity Assay of Trastuzumab, scFv thereof (Trastuzumab-scFv), and Trastuzumab-scFv-PPΔ Lacking Pro95 and Pro96)

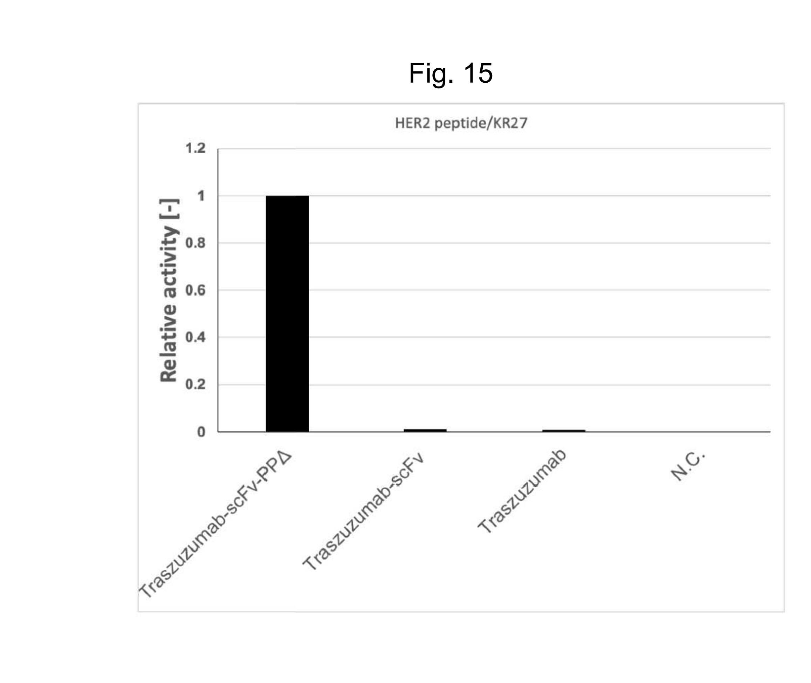

The antigen decomposition activity of Trastuzumab-scFv-PPΔ lacking Pro95 and Pro96 was significantly improved as compared with Trastuzumab and Trastuzumab-scFv (Figure 15).

Example 5 (Synthesis and Evaluation of Polypeptide (g))

The above mentioned polypeptide (g) is expressed using an expression vector comprising a polynucleotide encoding a polypeptide in which the polypeptide of the present invention consisting of the amino acid sequence set forth in SEQ ID NO: 6 and the polypeptide set forth in SEQ ID NO: 15 are linked to each other, by an expression system known in the art such as an intracellular or extracellular expression system.

Various activities of this polypeptide can be evaluated by comparing it with a control polynucleotide expressed by an expression system known in the art such as an intracellular or extracellular expression system using an expression vector comprising a polynucleotide encoding a polypeptide in which a polypeptide consisting of the amino acid sequence set forth in SEQ ID NO: 4 and a polypeptide set forth in SEQ ID NO: 15 are linked to each other.

[Sequence Listing]

SEQ ID NO: 1: RASQDVNTAVA (CDR1 region)
SEQ ID NO: 2: SASFLYS (CDR2 region)
SEQ ID NO: 3: QQHYTTPPT (CDR3 region)
SEQ ID NO: 4:

DIQMTQSPSSLSASVGDRVTITCRASQDVNTAVAWYQQKPGKAPKLLIYSASFLYSG

VPSRFSGSRSGTDFTLTISSLQPEDFATYYCQQHYTTPPTFGQGTKVEIKRTVAAPS

VFIFPPSDEQLKSGTASVVCLLNNFYPREAKVQWKVDNALQSGNSQESVTEQDSKDS

TYSLSSTLTLSKADYEKHKVYACEVTHQGLSSPVTKSFNRGEC

SEQ ID NO: 5: QQHYTTT (CDR3 region; ΔP95, 96)
SEQ ID NO: 6:
DIQMTQSPSSLSASVGDRVTITCRASQDVNTAVAWYQQKPGKAPKLLIYSASFLYSG VPSRFSGSRSGTDFTLTIS SLQPEDFATYYCQQHYTTTFGQGTKVEIKRTVAAPSVF IFPPSDEQLKSGTASVVCLLNNFYPREAKVQWKVDNA LQSGNSQESVTEQDSKDSTY SLSSTLTLSKADYEKHKVYACEVTHQGLSSPVTKSFNRGEC (sequence of the HER2 antibody light chain lacking Pro95 and Pro06)
SEQ ID NO: 7:

CCTACTACTGCCAGCAGCACTACACCACCCCGCCGACCTTCGGTCAGGGTACCAAAG

TTGAAATC

SEQ ID NO: 8:
ACC TTC GGT CAG GGT ACC AAA GTT GA (Forward primer)
SEQ ID NO: 9:
GGT GGT GTA GTG CTG CTG GCA GTA G (Reverse primer)
SEQ ID NO: 10:

DVVMTQTPLSLSVTPGEPASISCRSTQSLLDSDGVNPSFDWYVQKPGQSPQLLIHRG

FYRASGVPDRFSGSGSGTDFTLRISRVEAEDVGVYYCMQRIEFPLTFGGGTKVEIKR

TVAAPSVFIFPPSDEQLKSGTASVVCLLNNFYPREAKVQWKVDNALQSGNSQESVTE

QDSKDSTYSLSSTLTLSKADYEKHKLYACEVTHQGLSSPVTKSFNRGEC (amino

acid sequence of #4)

SEQ ID NO: 11:

DVVMTQTPLSLSVTPGEPASISCRSTQSLLDSDGVNPSFDWYVQKPGQSPQLLIHRG

FYRASGVPDRFSGSGSGTDFTLRISRVEAEDVGVYYCMQRIEFLTFGGGTKVEIKRT

VAAPSVFIFPPSDEQLKSGTASVVCLLNNFYPREAKVQWKVDNALQSGNSQESVTEQ

DSKDSTYSLSSTLTLSKADYEKHKLYACEVTHQGLSSPVTKSFNRGEC (amino

acid sequence of the polypeptide derived from #4 lacking

P95)

SEQ ID NO: 12:

EVQLVESGGGLVQPGGSLRLSCAASGFNIKDTYIHWVRQAPGKGLEWVARIYPTNGY

TRYADSVKGRFTISADTSKNTAYLQMNSLRAEDTAVYYCSRWGGDGFYAMDYWGQGT

LVTVSSASTKGPSVFPLAPSS (heavy-chain variable region)

SEQ ID NO: 13:
scFv sequence-1 (clone constructed by linking the anti-HER2 antibody light-chain variable region to the anti-HER2 antibody heavy-chain variable region via a linker)

MDIQMTQSPSSLSASVGDRVTITCRASQDVNTAVAWYQQKPGKAPKLLIYSASFLYS

GVPSRFSGSRSGTDFTLTISSLQPEDFATYYCQQHYTTPPTFGQGTKVEIKRGSGGG

GSGGGGSGGGGSKLEVQLVESGGGLVQPGGSLRLSCAASGFNIKDTYIHWVRQAPGK

GLEWVARIYPTNGYTRYADSVKGRFTISADTSKNTAYLQMNSLRAEDTAVYYCSRWG

GDGFYAMDYWGQGTLVTVSSASTKGPSVFPLAPSSLE

SEQ ID NO: 14:
scFv sequence-2
(clone constructed by linking the anti-HER2 antibody light-chain variable region lacking Pro96 and Pro96 in accordance with the Kabat numbering scheme to the anti-HER2 antibody heavy-chain variable region via a linker)

MDIQMTQSPSSLSASVGDRVTITCRASQDVNTAVAWYQQKPGKAPKLLIYSASFLYS

GVPSRFSGSRSGTDFTLTISSLQPEDFATYYCQQHYTTTFGQGTKVEIKRGSGGGGS

GGGGSGGGGSKLEVQLVESGGGLVQPGGSLRLSCAASGFNIKDTYIHWVRQAPGKGL

EWVARIYPTNGYTRYADSVKGRFTISADTSKNTAYLQMNSLRAEDTAVYYCSRWGGD

GFYAMDYWGQGTLVTVSSASTKGPSVFPLAPSSLE

SEQ ID NO: 15:
(amino acid sequence of the HER2 antibody heavy chain)

EVQLVESGGGLVQPGGSLRLSCAASGFNIKDTYIHWVRQAPGKGLEWVARIYPTNGY

TRYADSVKGRFTISADTSKNTAYLQMNSLRAEDTAVYYCSRWGGDGFYAMDYWGQGT

LVTVSSASTKGPSVFPLAPSSKSTSGGTAALGCLVKDYFPEPVTVSWNSGALTSGVH

TFPAVLQSSGLYSLSSVVTVPSSSLGTQTYICNVNHKPSNTKVDKKVEPKSCDKTHT

CPPCPAPELLGGPSVFLFPPKPKDTLMISRTPEVTCVVVDVSHEDPEVKFNWYVDGV

EVHNAKTKPREEQYNSTYRVVSVLTVLHQDWLNGKEYKCKVSNKALPAPIEKTISKA

KGQPREPQVYTLPPSREEMTKNQVSLTCLVKGFYPSDIAVEWESNGQPENNYKTTPP

VLDSDGSFFLYSKLTVDKSRWQQGNVFSCSVMHEALHNHYTQKSLSLSPGK

**Claims**

1.  A polypeptide having HER2 decomposition activity, wherein a hypervariable region is selected from the group consisting of (1a) to (1c):

    (1a) a polypeptide consisting of an amino acid sequence that includes a CDR1 region consisting of an amino acid sequence set forth in SEQ ID NO: 1, a CDR2 region consisting of an amino acid sequence set forth in SEQ ID NO: 2, and a CDR3 region consisting of an amino acid sequence set forth in SEQ ID NO: 3, and has an aspartic acid residue at position 70 of a variable region in accordance with the Kabat numbering scheme, wherein proline residues at positions 95 and 96 of the variable region in accordance with the Kabat numbering scheme are deleted or substituted with amino acid residues other than proline residues;
    (1b) a polypeptide consisting of an amino acid sequence that includes a CDR1 region consisting of an amino acid sequence in which one or several amino acids are substituted, added, or deleted in the amino acid sequence set forth in SEQ ID NO: 1, a CDR2 region consisting of an amino acid sequence in which one or several amino acids are substituted, added, or deleted in the amino acid sequence set forth in SEQ ID NO: 2, and a CDR3 region consisting of an amino acid sequence set forth in SEQ ID NO: 3, and has an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues; and
    (1c) a polypeptide consisting of an amino acid sequence that includes a CDR1 region consisting of an amino acid sequence having 90% or more identity with the amino acid sequence set forth in SEQ ID NO: 1, a CDR2 region consisting of an amino acid sequence having 90% or more identity with the amino acid sequence set forth in SEQ ID NO: 2, and a CDR3 region consisting of an amino acid sequence set forth in SEQ ID NO: 3, and has an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues.

2.  A polypeptide having HER2 decomposition activity, wherein a variable region is selected from the group consisting of (2a) to (2c):

    (2a) a polypeptide consisting of an amino acid sequence set forth in SEQ ID NO: 4;
    (2b) a polypeptide consisting of an amino acid sequence in which one or several amino acids are substituted, added, or deleted in the amino acid sequence set forth in SEQ ID NO: 4, the amino acid sequence having an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues; and
    (2c) a polypeptide consisting of an amino acid sequence having 90% or more identity with the amino acid sequence set forth in SEQ ID NO: 4, the amino acid sequence having an aspartic acid residue at position 70 of a variable region in accordance with the Kabat numbering scheme, wherein amino acid residues at positions 95 and 96 of the variable region in accordance with the Kabat numbering scheme are deleted or substituted with amino acid residues other than proline residues.

3. The polypeptide according to claim 1 or 2, further comprising a variable region of an anti-HER2 antibody heavy chain as set forth in SEQ ID NO: 12.

4. The polypeptide according to claim 3, wherein the hypervariable region of an anti-HER2 antibody light chain according to claim 1 and the variable region of an anti-HER2 antibody heavy chain as set forth in SEQ ID NO: 12, or the variable region of an anti-HER2 antibody light chain according to claim 2 and the variable region of an anti-HER2 antibody heavy chain as set forth in SEQ ID NO: 12, are linked via a linker.

5. The polypeptide according to claim 4, further comprising an Fc region linked thereto.

6. The polypeptide according to claim 3, wherein

a light-chain constant region linked to the hypervariable region of an anti-HER2 antibody according to claim 1 and a heavy-chain CH1 region linked to a heavy-chain variable region set forth in SEQ ID NO: 12 are associated through a disulfide bond; or
a light-chain constant region linked to the variable region of an anti-HER2 antibody according to claim 2 and a heavy-chain CH1 region linked to a heavy-chain variable region set forth in SEQ ID NO: 12 are associated through a disulfide bond.

7. A polypeptide comprising a heavy-chain CH2 and a heavy-chain CH3 that are linked to the CH1 region of the polypeptide according to claim 6.

8. A method for producing a polypeptide derived from an anti-HER2 antibody light chain having enzymatic activity or improved enzymatic activity, the method comprising:

a modification step of modifying a polynucleotide encoding an anti-HER2 antibody light chain in which a hypervariable region is (1a) a polypeptide comprising a CDR1 region consisting of an amino acid sequence set forth in SEQ ID NO: 1, a CDR2 region consisting of an amino acid sequence set forth in SEQ ID NO: 2, and a CDR3 region consisting of an amino acid sequence set forth in SEQ ID NO: 3, and having an aspartic acid residue at position 70 of a variable region in accordance with the Kabat numbering scheme; (1b) a polypeptide comprising a CDR1 region consisting of an amino acid sequence in which one or several amino acids are substituted, added, or deleted in the amino acid sequence set forth in SEQ ID NO: 1, a CDR2 region consisting of an amino acid sequence in which one or several amino acids are substituted, added, or deleted in the amino acid sequence set forth in SEQ ID NO: 2, and a CDR3 region consisting of an amino acid sequence set forth in SEQ ID NO: 3, and having an aspartic acid residue at position 70 of a variable region in accordance with the Kabat numbering scheme; or (1c) a polypeptide comprising a CDR1 region consisting of an amino acid sequence having 90% or more identity with the amino acid sequence set forth in SEQ ID NO: 1, a CDR2 region consisting of an amino acid sequence having 90% or more identity with the amino acid sequence set forth in SEQ ID NO: 2, and a CDR3 region consisting of an amino acid sequence set forth in SEQ ID NO: 3, and having an aspartic acid residue at position 70 of a variable region in accordance with the Kabat numbering scheme, wherein the polypeptide consists of an amino acid sequence in which amino acid residues at positions 95 and 96 of the variable region in accordance with the Kabat numbering scheme are proline residues, so that the proline residues are deleted or substituted to give a polynucleotide encoding a polypeptide derived from an anti-HER2 antibody light chain consisting of an amino acid sequence in which amino acid residues at positions 95 and 96 of the variable region in accordance with the Kabat numbering scheme are deleted or substituted with amino acid residues other than proline residues; and
an expression step of expressing a polypeptide derived from an anti-HER2 antibody light chain having enzymatic activity, by using an expression vector comprising a polynucleotide encoding an anti-HER2 antibody light chain after the modification step, in an intracellular or extracellular expression system.

9. A method for producing a polypeptide derived from an anti-HER2 antibody light chain having enzymatic activity or improved enzymatic activity, the method comprising:

a modification step of modifying a polynucleotide encoding a polypeptide derived from an anti-HER2 antibody light chain in which a variable region is (2a) a polypeptide consisting of an amino acid sequence set forth in SEQ ID NO: 4; (2b) a polypeptide consisting of an amino acid sequence in which one or several amino acids are substituted, added, or deleted in the amino acid sequence set forth in SEQ ID NO: 4, the amino acid sequence having an aspartic acid residue at position 70 of a variable region in accordance with the Kabat numbering scheme; or (2c) a polypeptide having 90% or more identity with the amino acid sequence set forth in SEQ ID NO: 4 and having an aspartic acid residue at position 70 of a variable region in accordance with the Kabat numbering scheme, wherein the polypeptide consists of an amino acid sequence in which amino acid residues at positions 95 and 96 of the variable region in accordance with the Kabat numbering scheme are proline residues, so that the proline residues are deleted or substituted to give a polynucleotide encoding a polypeptide derived from an anti-HER2 antibody light chain, wherein the variable region is a polypeptide consisting of an amino acid sequence in which amino acid residues at positions 95 and 96 of the variable region in accordance with the Kabat numbering scheme are deleted or substituted with amino acid residues other than proline residues; and

an expression step of expressing a polypeptide derived from an anti-HER2 antibody light chain having enzymatic activity, by using an expression vector comprising a polynucleotide encoding an anti-HER2 antibody light chain after the modification step, in an intracellular or extracellular expression system.

10. An anticancer agent comprising a component selected from the group consisting of: the polypeptide derived from an anti-HER2 antibody light chain according to claim 1 or 2; a HER2-binding polypeptide comprising the polypeptide derived from the light chain; and a functional fragment of the HER2-binding polypeptide comprising the polypeptide derived from the light chain.

11. A pharmaceutical composition for treating cancer, the pharmaceutical composition comprising a component selected from the group consisting of: the polypeptide derived from an anti-HER2 antibody light chain according to claim 1 or 2; a HER2-binding polypeptide comprising the polypeptide derived from the light chain; and a functional fragment of the HER2-binding polypeptide comprising the polypeptide derived from the light chain.

12. Use of a component for production of an anticancer agent, the component being selected from the group consisting of: the polypeptide derived from an anti-HER2 antibody light chain according to claim 1 or 2; a HER2-binding polypeptide comprising the polypeptide derived from the light chain; and a functional fragment of the HER2-binding polypeptide comprising the polypeptide derived from the light chain.

13. A component for use in treatment of cancer, the component being selected from the group consisting of: the polypeptide derived from an anti-HER2 antibody light chain according to claim 1 or 2; a HER2-binding polypeptide comprising the polypeptide derived from the light chain; and a functional fragment of an anti-HER2 antibody comprising the polypeptide derived from the light chain.

14. A method for treating cancer, the method comprising administering a component selected from the group consisting of: the polypeptide derived from an anti-HER2 antibody light chain according to claim 1 or 2; a HER2-binding polypeptide comprising the polypeptide derived from the light chain; and a functional fragment of the HER2-binding polypeptide comprising the polypeptide derived from the light chain.

# Fig. 1

P95P96

CCTACTACTGCCAGCAGCACTACACCACCCCGCCGACCTTCGGTCAGGG

TACCAAAGTTGAAATC

(SEQ ID NO: 7)

# Fig. 2A

Trastuzumab L

Fig. 2B

**B)**

reduced | non-reduced

(kDa) | M
97.4
66.2
45.0
31.0
21.5
14.4

**Trastuzumab LΔΔ**

Fig. 2C

scFv Trastuzumab LΔΔ

# Fig. 3

R-pNA

Trastuzumab _light chain P95Δ_P96Δ

scFv Trastuzumab_P95Δ_P96Δ

Trastuzumab_Light chain

pNA concentration (μM)

Reaction time (h)

Experimental condition
Substrate concentration: 200 μM
Abzyme: 4.3 μM

N=3, 100uL/well, 37°C

# Fig. 4

**a)** FRET-Her2 peptide-1 (579-600)

K(MCA)-PSGVKPDLSYMPIWKFPDEEGA-K(DNP)-rrr-NH2

**b)** FRET-Her2 peptide-2 (Loop2 + Loop3)

K(MCA)-AHYKDPPFGGSYMPIWKFPDEEGA-K(DNP)-rrr-NH2

EP 4 737 480 A1

EP 4 737 480 A1

# Fig. 5

Cleaved site of FRET-HER2-1 peptide

K(MCA)-PSGVKPDLSYMPIWK FPDEEGA-K(DNP)-rrr-NH$_2$(DNP)

Cleaved site

FRET-HER2-I alone

K(MCA)-PSGVKPDLSYMPIW**K**

FPDEEGA-K(DNP)-rrr-NH$_2$(DNP)

VL
P95 · 96Δ
CL

FRET-HER2-I +
Tmb-L-PPΔ, 50 µL injection

VL
P95 · P96Δ
VH

FRET-HER2-I +
Tmb-scFv-PPΔ, 20 µL injection

FPDEEGA-K(DNP)-rrr-NH$_2$(DNP)

20.00  22.00  24.00  26.00  28.00  30.00  32.00  34.00  36.00  38.00  40.00 min

KR27
K(MCA)-PSGVKPDLSYMPIWK-FPDEEGA-K(DNP)-rrr-NH2

column : Cosmosil type : 5C$_{18}$-AR-2 (4.6×250), MilliQ water in 0.05 % TFA : Acetonitrile in 0.05 % TFA from 90 : 10 to 30 : 70 in 60 min

Fig. 6

KR27/Peptide-1

Fig. 7

CDR1
```
    1            10            20    24        30        35        40
DIQMTQSPSSLSASVGDRVTITCRASQDVNTAVAWYQQKPGKA
```

CDR2
```
44         50          57  60              70            80
PKLLIYSASFLYSGVPSRFSGSRSGTDFTLTISSLQPEDFATY
```

CDR3
```
87 89       95  98 100          110           120
YCQQHYTTPPTFGQGTKVEIKRTVAAPSVFIFPPSDEQLKSGT
```

```
130         140           150           160           170
ASVVCLLNNFYPREAKVQWKVDNALQSGNSQESVTEQDSKDST
```

```
173    180          190           200           210
YSLSSTLTLSKADYEKHKVYACEVTHQGLSSPVTKSFNRGEC
```

# Fig. 8

Cleaved site of FRET-HER2-2 peptide

K(MCA)-AHYKDPPPF-gg-SYMPIWK FPDEEGA-K(DNP)-rrr-NH$_2$(DNP)→

Cleaved site

FRET-HER2 peptide-2 alone

VL
P95·96Δ
CL

FRET-HER2 peptide-2 +
Tmb-L-PPΔ, 40 μL injection

K(MCA)-AHYKDPPPF-gg-SYMPIWK

VL
P95·P96Δ
VH

FRET-HER2 peptide-2 +
Tmb-scFv-PPΔ, 55 μL injection

K(MCA)-AHYKDPPPF-gg-SYMPIWK

20.00  22.00  24.00  26.00  28.00  30.00  32.00  34.00  36.00  38.00  40.00 min

KR29
K(MCA)-AHYKDPPFGG SYMPIWK-FPDEEGA-K(DNP)-rrr-NH2

column : Cosmosil type : 5C$_{18}$-AR-2 (4.6×250), MilliQ water in 0.05 % TFA : Acetonitrile in 0.05 % TFA from 90 : 10 to 30 : 70 in 60 min

Fig. 9

# Fig. 10

**Kinetic constants of L-PPΔ and scFv-PPΔ**
**Lineweaver–Burk plot**

a)

VL
P95 · 96Δ
CL

$y = 115.2x + 3.8965$
$R^2 = 0.9383$

1/v (hr/μM)

1/[S] (/μM)

kcat = 0.85 x $10^{-3}$ $min^{-1}$
Km = 2.95 x $10^{-5}$ M
kcat/Km = 28.8 $M^{-1}$ $min^{-1}$

b)

VL
P95 · P96Δ
VH

$y = 191.22x + 3.4575$
$R^2 = 0.9816$

1/v (hr/μM)

1/[S] (/μM)

kcat = 0.96 x $10^{-3}$ $min^{-1}$
Km = 5.53 x $10^{-5}$ M
kcat/Km = 17.4 $M^{-1}$ $min^{-1}$

## Fig. 11

CDR1

DVVMTQTPLSLSVTPGEPASISCRSTQSLLDSDGVNPSFDWYV

CDR2

QKPGQSPQLLIHRGFYRASGVPDRFSGSGSGTDFTLRISRVEA

CDR3

EDVGVYYCMQRIEFPLTFGGGTKVEIKRTVAAPSVFIFPPSDE

QLKSGTASVVCLLNNFYPREAKVQWKVDNALQSGNSQESVTEQ

DSKDSTYSLSSTLTLSKADYEKHKLYACEVTHQGLSSPVTKSF

NRGEC

Fig. 12

Fig. 13

EP 4 737 480 A1

Fig. 14

EP 4 737 480 A1

## Fig. 15

HER2 peptide/KR27

## INTERNATIONAL SEARCH REPORT

| International application No. |
|---|
| **PCT/JP2024/023474** |

**A. CLASSIFICATION OF SUBJECT MATTER**

*C07K 16/28*(2006.01)i; *A61K 39/395*(2006.01)i; *A61K 47/64*(2017.01)i; *A61P 35/00*(2006.01)i; *A61P 43/00*(2006.01)i; *C12N 9/50*(2006.01)i; *C12N 15/13*(2006.01)i; *C12N 15/57*(2006.01)i; *C12P 21/08*(2006.01)i

FI:  C07K16/28; C12P21/08; A61P43/00 111; A61P35/00; A61K39/395 E; A61K39/395 T; A61K47/64; A61K39/395 V; C12N15/13; C12N15/57; C12N9/50 ZNA

According to International Patent Classification (IPC) or to both national classification and IPC

**B. FIELDS SEARCHED**

Minimum documentation searched (classification system followed by classification symbols)

C07K16/28; A61K39/395; A61K47/64; A61P35/00; A61P43/00; C12N9/50; C12N15/13; C12N15/57; C12P21/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); GenBank/EMBL/DDBJ/GeneSeq; UniProt/GeneSeq

**C. DOCUMENTS CONSIDERED TO BE RELEVANT**

| Category* | Citation of document, with indication, where appropriate, of the relevant passages | Relevant to claim No. |
|---|---|---|
| A | JP 2017-519759 A (MERSANA THERAPEUTICS, INC.) 20 July 2017 (2017-07-20) examples, fig. 8 | 1-14 |
| A | WO 2021/015237 A1 (JAPAN SCIENCE AND TECHNOLOGY AGENCY) 28 January 2021 (2021-01-28) claims, examples, fig. 5B | 1-14 |

☑ Further documents are listed in the continuation of Box C.       ☑ See patent family annex.

| | | |
|---|---|---|
| \* | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **16 August 2024** | **17 September 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT

| | International application No. |
|---|---|
| | **PCT/JP2024/023474** |

**C.      DOCUMENTS CONSIDERED TO BE RELEVANT**

| Category* | Citation of document, with indication, where appropriate, of the relevant passages | Relevant to claim No. |
|---|---|---|
| A | 宇田　泰三,「スーパー抗体酵素」の酵素活性発現メカニズムの解明と効率的作製法, 戦略的創造研究推進事業　ＣＲＥＳＴ　研究領域「医療に向けた化学・生物系分子を利用したバイオ素子・システムの創製」　研究課題「健康・福祉のためのナノバイオ材料およびバイオ素子としての「スーパー抗体酵素」の創製」　研究終了報告書, 20 July 2020, pp. 24-37, non-official translation (UDA, Taizo. Elucidation of the mechanism of enzyme activity expression of 'super antibody enzyme' and efficient production method. JST Strategic Basic Research Programs CREST. Research area: "Creation of bio element and systems using chemical and biological molecules towards medical care". Research topic: "Creation of 'super antibody enzymes' as nanobiomaterials and bio element for health and welfare". Research Final Report.)<br>    pp. 24-37, fig. 21 | 1-14 |
| A | CARTER, Paul et al. Humanization of an anti-p185HER2 antibody for human cancer therapy. Proc.Natl.Acad.Sci.USA. 1992, vol. 89, pp. 4285-4289<br>    fig. 1 | 1-14 |

Form PCT/ISA/210 (second sheet) (July 2022)

**INTERNATIONAL SEARCH REPORT**

International application No.

**PCT/JP2024/023474**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/JP2024/023474**

| Patent document cited in search report | | | Publication date (day/month/year) | Patent family member(s) | | | Publication date (day/month/year) |
|---|---|---|---|---|---|---|---|
| JP | 2017-519759 | A | 20 July 2017 | WO 2015/195917 A1 examples, fig. 8 | | | |
| WO | 2021/015237 | A1 | 28 January 2021 | US 2022/0259290 A1 claims, examples, fig. 5B | | | |

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006197930 A
- WO 2011102517 A
- WO 2013133253 A
- WO 2015025786 A
- WO 2021015237 A

**Non-patent literature cited in the description**

- Remington's Pharmaceutical Sciences. Mack Publishing Co, 1985